# EUROPEAN PATENT SPECIFICATION

(11) **EP 2 033 934 B1**
(45) Date of publication and mention of the grant of the patent: **28.09.2011**
(21) Application number: 07744995.7
(22) Date of filing: 11.06.2007
(51) Int. Cl.: D01D 5/24, B82Y 5/00, A61K 9/00, A61K 47/48, C07H 13/06

(54) **HOLLOW-FIBER-LIKE ORGANIC NANOTUBE AND PROCESS FOR PRODUCTION THEREOF**
HOHLFASERFÖRMIGES ORGANISCHES NANOROHR UND VERFAHREN ZU SEINER HERSTELLUNG
NANOTUBE ORGANIQUE EN FORME DE FIBRE CREUSE ET SON PROCÉDÉ DE FABRICATION

(30) Priority: 14.06.2006 JP 2006164269; 26.06.2006 JP 2006174713; 23.05.2007 JP 2007136277
(43) Date of publication of application: 11.03.2009
(73) Proprietor: National Institute of Advanced Industrial Science and Technology, Tokyo 100-8921 (JP)
(72) Inventor: ASAKAWA, Masumi, Tsukuba-shi Ibaraki 305-8565 (JP); KOGISO, Masaki, Tsukuba-shi Ibaraki 305-8565 (JP); KAMIYA, Shouko, Tsukuba-shi Ibaraki 305-0045 (JP); SIMIZU, Toshimi, Tsukuba-shi Ibaraki 305-8565 (JP)
(74) Representative: Daniels, Jeffrey Nicholas
(86) International application number: PCT/JP2007/061703
(87) International publication number: WO 2007/145158

(56) References cited:
- EP-A1- 1 609 796
- WO-A1-2005/082918
- JP-A- 11 255 791
- JP-A- 2004 224 717
- JP-A- 2004 250 797
- JP-A- 2004 256 414
- JP-A- 2004 261 885
- JP-A- 2004 261 885
- JP-A- 2005 060 326
- KAMIYA M. ET AL.: 'Amide-gata Toshishitsu Nanotube no Keitai ni Ataeru Chosei Joken no Eikyo' CSJ: THE CHEMICAL SOCIETY OF JAPAN KOEN YOKOSHU vol. 86TH, no. 1, 13 March 2006, page 652, XP003020601
- DIAZ N. ET AL.: 'Self-Assembled Diamide Nanotubes in Organic Solvents' ANGEWANDTE CHEMIE. INTERNATIONAL EDITION vol. 44, no. 21, 20 May 2005, pages 3260 - 3264, XP003020602

## Description

### BACKGROUND

### 1. Technical Field

The present invention relates to a method of producing a water-free organic nanotube useful as a highly-functional material, such as an encapsulating/separating material or a sustained drug release material in the field of medical/chemical products, in a simple manner and in large quantities.

### 2. Related Art

Among nanotubular materials having a pore with a size of 0.5 to 500 nm, a carbon nanotube is an inorganic nanotube which was artificially synthesized for the first time. With expectations for features of the carbon nanotube, such as size, configuration and chemical structure, studies on applications to nanoscale electronic devices, high-strength materials, electron emission and gas storage, and researches on mass production techniques toward practical use thereof, have been strenuously carried out (see, for example, the following Patent Document 1).

Cyclodextrin is known as an organic cyclic compound having a pore with a size of 1 nm or less, which is capable of encapsulating various low-molecular organic compounds in an annular hollow space thereof. Thus, through the research and development for contribution to the fields of health foods, cosmetic products, household antibacterial/deodorant products and industry/agriculture/environment, various cyclodextrin-encapsulated products have been commercialized (see, for example, the following Patent Document 2). Cyclodextrin can be produce in large quantities, and biological safety thereof is ensured because its structure is made up of annularly linked 6 to 8 glucose units. Thus, a wide range of application is being studied.

The inventors of this application have already developed an organic nanotube obtainable through self-assembling in an aqueous solution (see, for example, the following Patent Document 3 and Non-Patent Documents 1 to 3). This organic nanotube developed by the inventors is a hollow cylinder having an inner pore with a size of 5 to 500 nm which is one digit greater than that of cyclodextrin. Thus, it has a potential to be able to capture a functional substance, such as protein, virus, chemicals or metal nanoparticles, within the hollow cylinder, and therefore there are great expectations to utilize the features for various purposes.

The conventional organic nanotube has been synthesized in an aqueous solution. Thus, in a production process, it is necessary to use a large volume of water, and perform an operation of heating and stirring the solution and an operation of maintaining the solution in a stationary state for a long period of time, which causes difficulty in ensuring mass-productivity. Moreover, the organic nanotube synthesized in an aqueous solution strongly holds water in its structure (this organic nanotube will hereinafter be referred to as "water-containing organic nanotube"), and the water is hardly removed by conventional methods, which causes a problem of being unable to efficiently encapsulate a functional substance into the water-containing organic nanotube.

| | |
|---|---|
| [Patent Document 1] | JP 2003-535794A |
| [Patent Document 2] | JP 2005-306763A |
| [Patent Document 3] | JP 3 664401 |
| [Non-Patent Document 1] | Langmuir, 2005, 21, 743 |
| [Non-Patent Document 2] | Chem. Comm., 2004, 500 |
| [Non-Patent Document 3] | Chem. Mater., 2004, 16, 2826 |

### SUMMARY OF THE INVENTION

In view of the above problems in the conventional water-containing organic nanotube, it is an object of the present invention to provide a method of synthesizing an organic nanotube containing no water (hereinafter referred to as "water-free organic nanotube") capable of efficiently encapsulating a functional substance therein.

Through various researches for solving the above problems, the inventors found that a water-free organic nanotube can be produced in a simple manner and in large quantities by allowing an N-glycoside type glycolipid or a peptide lipid to self-assemble in an organic solvent, instead of water. Based on this knowledge, the inventors have finally accomplished the present invention.

Specifically, the present invention provides a method of producing a water-free organic nanotube, which comprises the steps of: dissolving, in an organic solvent heated up to a temperature equal to or less than a boiling point thereof, one selected from the group consisting of an N-glycoside type glycolipid represented by the following general formula 1: G-NHCO-R¹ (wherein G represents a sugar residue from which a hemiacetal hydroxyl group bonded to an anomeric carbon atom of the sugar is excluded; and R¹ represents an unsaturated hydrocarbon group having 10 to 39 carbon atoms), a first peptide-lipid conjugate represented by the following general formula 2: R²CO (NH-CHR³-CO)ₘ OH (wherein: R² represents a hydrocarbon group having 6 to 24 carbon atoms; R³ represents an amino-acid side chain; and m represents an integer of 1 to 10), and a second peptide-lipid conjugate represented by the following general formula 3: H (NH-CHR³-CO)ₘ NHR² (wherein: R² represents a hydrocarbon group having 6 to 24 carbon atoms; R³ represents an amino-acid side chain; and m represents an integer of 1 to 10); slowly cooling the obtained solution; maintaining the cooled solution undisturbed at room temperature; and collecting from the resulting solution a hollow fibrous organic nanotube formed through self-assembling in the stationary solution, and drying the collected hollow fibrous organic nanotube, in air at room temperature or by heating under reduced-pressure.

The present invention also provides a method of producing a water-free organic nanotube, comprising the steps of: dissolving in an organic solvent one selected from the group consisting of an N-glycoside type glycolipid represented by the following general formula 1: G-NHCO-R¹ (wherein G represents a sugar residue from which a hemiacetal hydroxyl group bonded to an anomeric carbon atom of the sugar is excluded; and R¹ represents an unsaturated hydrocarbon group having 10 to 39 carbon atoms), a first peptide-lipid conjugate represented by the following general formula 2: R²CO (NH-CHR³-CO)ₘ OH (wherein: R² represents a hydrocarbon group having 6 to 24 carbon atoms; R³ represents an amino-acid side chain; and m represents an integer of 1 to 10), and a second peptide-lipid conjugate represented by the following general formula 3: H (NH-CHR³-CO)ₘ NHR² (wherein: R² represents a hydrocarbon group having 6 to 24 carbon atoms; R³ represents an amino-acid side chain; and m represents an integer of 1 to 10); concentrating the obtained solution: maintaining the concentrated solution undisturbed at room temperature; and collecting from the resulting solution a hollow fibrous organic nanotube formed through self assembling in the stationary solution, and drying the collected hollow fibrous organic nanotube, in air at room temperature or by heating under reduced-pressure.

Further, the present invention provides a method of producing a hollow fibrous organic nanotube, which comprises the steps of: dissolving, in an organic solvent heated up to a temperature equal to or less than a boiling point thereof, one selected from the group consisting of an N-glycoside type glycolipid represented by the following general formula 1: G-NHCO-R¹ (wherein G represents a sugar residue from which a hemiacetal hydroxyl group bonded to an anomeric carbon atom of the sugar is excluded; and R¹ represents an unsaturated hydrocarbon group having 10 to 39 carbon atoms), a first peptide-lipid conjugate represented by the following general formula 2: R²CO (NH-CHR³-CO)ₘ OH (wherein: R² represents a hydrocarbon group having 6 to 24 carbon atoms; R³ represents an amino-acid side chain; and m represents an integer of 1 to 10), and a second peptide-lipid conjugate represented by the following general formula 3: H (NH-CHR³-CO)ₘ NHR² (wherein: R² represents a hydrocarbon group having 6 to 24 carbon atoms; R³ represents an amino-acid side chain; and m represents an integer of 1 to 10); adding to the obtained solution a poor solvent for the N-glycoside type glycolipid or the first and second peptide-lipid conjugates; maintaining the mixed solution undisturbed at room temperature; and collecting from the resulting solution a hollow fibrous organic nanotube formed through self-assembling in the stationary solution, and drying the collected hollow fibrous organic nanotube, in air at room temperature or by heating under reduced-pressure.

The water-free organic nanotube produced by the method of the present invention contains no water in a tubular film structure or inside a hollow cylinder, and allows the organic solvent other than water to be fully removed therefrom. Thus, the water-free organic nanotube of the present invention can readily encapsulate a functional substance therein. In addition, the water-free organic nanotube of the present invention can be efficiently produced as compared with the conventional hollow fibrous organic nanotube which was produced in an aqueous solution.

### BRIEF DESCRIPTION OF THE DRAWINGS

FIG. 1 is a photograph showing a scanning transmission electron microscopic (STEM) observation image of organic nanotubes obtained in Example 1.

FIG. 2 is a photograph showing a scanning transmission electron microscopic (STEM) observation image of organic nanotubes obtained in Example 2.

FIG 3 is a photograph showing a scanning transmission electron microscopic (STEM) observation image of organic nanotubes obtained in Example 3.

FIG. 4 is a photograph showing a scanning electron microscopic (SEM) observation image of organic nanotubes obtained in Example 4.

FIG. 5 is a photograph showing a scanning electron microscopic (SEM) observation image of an organic nanotube obtained in Example 5.

FIG. 6 is a photograph showing a scanning electron microscopic (SEM) observation image of organic nanotubes obtained in Example 6.

FIG 7 is a photograph showing a scanning electron microscopic (SEM) observation image of organic nanotubes obtained in Example 7.

FIG. 8 is a photograph showing a scanning electron microscopic (SEM) observation image of organic nanotubes obtained in Example 8.

FIG. 9 is a photograph showing a scanning electron microscopic (SEM) observation image of organic nanotubes obtained in Example 9.

FIG. 10 is a photograph showing a scanning electron microscopic (SEM) observation image of organic nanotubes obtained in Example 10.

FIG. 11 is a photograph showing a scanning electron microscopic (SEM) observation image of organic nanotubes obtained in Example 11.

FIG 12 is a graph showing a differential scanning calorimetry curve of a water-free organic nanotube (Example 2).

FIG. 13 is a graph showing a differential scanning calorimetry curve of a water-containing organic nanotube (Comparative Example 1).

FIG. 14 is a graph showing a powder X-ray diffraction pattern of water-containing organic nanotubes (Comparative Example 1).

FIG. 15 is a graph showing a powder X-ray diffraction pattern of water-free organic nanotubes (Example 2).

FIG. 16 is a graph showing time-series infrared absorption spectra of a water-free organic nanotube (Example 2).

FIG 17 is a graph showing a temporal intensity change in time-series infrared absorption spectra of a water-free organic nanotube (Example 2).

FIG 18 is a graph showing time-series infrared absorption spectra of a water-containing organic nanotube (Comparative Example 1).

FIG. 19 is a graph showing a temporal intensity change in time-series infrared absorption spectra of a water-containing organic nanotube (Comparative Example 1).

FIG 20 is a graph showing infrared absorption spectra of a water-free organic nanotube (Example 2) and a water-containing organic nanotube (Comparative Example 1), each after drying.

FIG. 21 is a graph showing respective infrared absorption spectra of three types of water-free organic nanotubes (Examples 1 to 3) after removal of organic solvent components.

FIG 22 is a transmission electron microscopic (TEM) photograph of a water-free organic nanotube having gold particles encapsulated therein (Example 12).

FIG. 23 is a transmission electron microscopic (TEM) photograph of a water-containing organic nanotube having gold particles encapsulated therein (Comparative Example 2).

FIG 24 is a transmission electron microscopic (TEM) photograph of a water-free organic nanotube having ferrosoferric oxide nanoparticles encapsulated therein (Example 13).

FIG. 25 is a transmission electron microscopic (TEM) photograph of a water-free organic nanotube having an iron storage protein, ferritin, encapsulated therein (Example 14), wherein a spectrum on a lower left side thereof is based on energy dispersive X-ray (EDX) analysis.

FIG. 26 is a photograph showing a scanning transmission electron microscopic (STEM) observation image of a water-free organic nanotube having gold nanoparticles encapsulated therein (Example 15).

### DETAILED DESCRIPTION OF THE EXEMPLARY EMBODIMENTS

SYNTHESIS OF N-GLYCOSIDE TYPE GLYCOLIPID

An N-glycoside type glycolipid in the present invention and a synthesis process therefor will be described.

An N-glycoside type glycolipid in the present invention has an unsaturated hydrocarbon group as an aglycone, and is represented by the following general formula 1: G-NHCO-R¹. This N-glycoside type glycolipid can be used as a raw material for producing a water-free organic nanotube.

In the general formula 1, G represents a sugar residue from which a hemiacetal hydroxyl group bonded to an anomeric carbon atom of the sugar is excluded. This sugar may be a glucose, galactose, maltose, lactose, cellobiose or chitobiose. Preferably, the sugar is glucopyranose. The sugar is a monosaccharide or an oligosaccharide, preferably a monosaccharide. The sugar residue may be in any one of the D-form, L-form and racemic form. Generally, a naturally-occurring sugar residue is in the D-form. An anomeric carbon atom is an asymmetrical carbon atom in an aldopyranosyl group, and therefore an α-anomer and a β-anomer exist therein. However, any one of an α-anomer, a β-anomer or a mixture thereof may be used. In particular, a sugar having a D-glucopyranosyl group or a D-galactopyranosyl group, particularly of a D-glucopyranosyl group, as the sugar residue G, is preferable in view of its advantage of high availability of a raw material, and high manufacturability.

In the general formula 1, R¹ is an unsaturated hydrocarbon group which preferably has a straight-chain structure, and, more preferably, having three or fewer double bonds as unsaturated bond. The number of carbon atoms in the unsaturated hydrocarbon group R is 10 to 39, preferably 15 to 20, more preferably 17. The unsaturated hydrocarbon group R may be a type in which a monoene, a diene or a triene portion is introduced as an unsaturated bond, in any one of an undecyl group, a dodecyl group, a tridecyl group, a tetradecyl group, a pentadecyl group, a hexadecyl group, a heptadecyl group, an octadecyl group, a nonadecyl group, an eicosyl group, a heneicosyl group, a docosyl group, a tricosyl group, a tetracosyl group, a pentacosyl group, a hexacosyl group, a heptacosyl group and an octacosyl group.

A production process for the N-glycoside type glycolipid in the present invention is not particularly limited. For example, the N-glycoside type glycolipid can be produced by allowing an unsaturated long-chain carboxylic acid represented by the following general formula: R¹-COOH (wherein R¹ represents the same as R¹ in the general formula 1) or an unsaturated long-chain carboxylic acid chloride represented by the following general formula: R¹-COCl (wherein R¹ represents the same as R¹ in the general formula 1) to react with a glycoamine having an amino group at an anomeric position so as to form an N-glycoside bond.

A production process for the glycoamine is not particularly limited. For example, the glycoamine can be produced in the following manner.

A specific sugar (aldopyranose or oligosaccharide thereof) and ammonium hydrogen carbonate are dissolved in water. Preferably, a temperature of this aqueous solution is set at 37°C. Consequently, the ammonium hydrogen carbonate selectively reacts with a hemiacetal hydroxyl group bonded to an anomeric carbon atom of the sugar to form an aminated product having an amino group at an anomeric carbon atom (C-1) of the sugar, i.e., so-called "amino sugar". In this reaction, a β-unit is selectively obtained.

The unsaturated long-chain carboxylic acid may include 10-undecenoic acid, 11-dodecenoic acid, 12-tridecenoic acid, 9-tetradecenoic acid, 10-pentadecenoic acid, 9-hexadecenoic acid, 10-heptadecenoic acid, 6-octadecenoic acid, 9-octadecenoic acid, 11-octadecenoic acid, 9-12 octadecadienoic acid, 6-9-12 octadecatrienoic acid, 9-12-15 octadecatrienoic acid, 7-nonadecenoic acid, 10-nonadecenoic acid, 10-13 nonadecadienoic acid, 5-eicosenoic acid, 8-eicosenoic acid, 11- eicosenoic acid, 11-14 eicosadienoic acid, 8-11-14 eicosatrienoic acid, 11-14-17 eicosatrienoic acid, heneicosenoic acid, 13-docosenoic acid, 13-16 docosadienoic acid, 13-16-19 docosatrienoic acid, 14-tricosenoic acid, 15-tetracosenoic acid, 10-12 tricosadiynoic acid, 10-12 pentacosadiynoic acid, 10-12 heptacosadiynoic acid, and 10-12 nonacosadiynoic acid. Among them, 11-octadecenoic acid is preferable in view of its advantage of good balance of amphiphilicity provided to the resulting glycolipid, and a melting point in water.

In the reaction between the unsaturated long-chain carboxylic acid and the glycoamine, a condensation agent may be used as a reaction accelerator. For example, this condensation agent may include benzotriazol-1-yl-oxy-tris(dimethylamino)-phosphonium hexafluoro phosphate (hereinafter referred to as "BOP", 1-hydroxybenzotriazole (hereinafter referred to as "HOBt", benzotriazol-1-yl-oxy-tripyrrolidinophosphonium hexafluoro phosphate (hereinafter referred to as "PyBOP"), O-(benzotriazol-1-yl)-N,N,N',N'-tetramethyluronium hexafluoro phosphate (hereinafter referred to as "HBTU"), diphenyl(2,3-dihydro-2-thioxo-3-benzoxazolyl) phosphonate (hereinafter referred to as "DBP"). Among them, a combination of BOP and HOBt is preferable because it induces reaction in good yield.

The operation of causing the reaction between the glycoamine and the unsaturated long-chain carboxylic acid to form an N-glycoside bond may be performed as follows.

The glycoamine and the unsaturated long-chain carboxylic acid are stirred at room temperature for 5 hours or more with a magnetic stirrer, using the combination of HOBt and BOP as a condensation agent and dimethylsulfoxide as a solvent. In order to enhance a yield, a mole ratio of fatty acid : glycoamine : HOBt : BOP is set to be 1 : 1 to 100 : 1 to 100 : 1 to 100, particularly 1 : 2 or more : 1 : 3. That is, each of the glycoamine and the condensation agent is excessively added to a fatty acid.

A bonding agent other than the combination of HOBt and BOP may include DBP itself, a combination of HOBt and HBTU, a combination of HOBt and PyBOP, a combination of DBP and triethylamine (TEA), and a combination of HOBt, HBTU and N,N-diisopropylethylamine (DIEA).

Preferably, the fatty acid and the condensation agent are mixed in a solvent and stirred for about 10 minutes to activate a carboxylic acid of the fatty acid before the reaction, and then the glycoamine is added thereto. After completion of the reaction, a resulting crude product can be highly refined through a separation and refinement process using a silica gel column and a ToyoPearl HW-40S column (produced by Tosoh Corporation)

The unsaturated long-chain carboxylic acid chloride may include oleoyl chloride, cis-vaccenoyl chloride, and linoleoyl chloride.

The reaction to form an N-glycoside bond from the glycoamine and the unsaturated long-chain carboxylic acid chloride may be performed as follows.

The glycoamine and the unsaturated long-chain carboxylic acid chloride are stirred with a magnetic stirrer at a reaction temperature of 0°C for 5 hours or more in a presence of a basic substance. A solvent may include methanol, water and tetrahydrofuran. Preferably, the glycoamine and a basic substance are mixed in a solvent and stirred at 0°C for about 2 hours to activate an amino group of the glycoamine before the reaction, and then the unsaturated long-chain carboxylic acid chloride is added thereto.

As a result of the above reaction, the N-glycoside type glycolipid in the present invention is formed in such a manner that an unsaturated hydrocarbon group is bonded to an anomeric carbon atom (C-1) of the sugar through an amide bond. A structure of the formed N-glycoside type glycolipid can be checked by a ¹H-NMR spectrum and a ¹³C-NMR spectrum.

SYNTHESIS OF PEPTIDE-LIPID CONJUGATE

A peptide-lipid conjugate in the present invention and a synthesis process will be described.

The peptide-lipid conjugate in the present invention is a peptide-lipid conjugate having a long-chain hydrocarbon group, which is represented by the following general formula 2: R²CO (NH-CHR³-CO)ₘ OH (this peptide-lipid conjugate will hereinafter be referred to as "first peptide-lipid conjugate"), or by the following general formula 3: H (NH-CHR³Y-CO)ₘ NHR² (this peptide-lipid conjugate will hereinafter be referred to as "second peptide-lipid conjugate"). This first or second peptide-lipid conjugate can be used as a raw material for producing a water-free organic nanotube.

In the general formula 2 and the general formula 3, R² is a hydrocarbon group having 6 to 24 carbon atoms, and preferably a straight-chain hydrocarbon which may have a side chain with two carbons or less. This hydrocarbon group may be a saturated group or may be an unsaturated group. The number of carbons in the hydrocarbon group R is 6 to 24, preferably 10 to 16. The hydrocarbon group may include hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, eicosyl group, heneicosyl group, docosyl group, tricosyl group, tetracosyl group, pentacosyl group and hexacosyl group.

In the general formula 2 and the general formula 3, R³ is an amino-acid side chain. This amino acid may include glycine, valine, leucine, isoleucine, alanine, arginine, glutamine, lysine, asparagine acid, glutamine acid, proline, cysteine, threonine, methionine, histidine, phenylalanine, tyrosine, triptophan, asparagine and serine. Preferably, the amino acid is glycine. The amino-acid side chain may be any one of the D-form, L-form and racemic form. Generally, a naturally-occurring amino-acid side chain is in the L-form.

In the general formula 2 and the general formula 3, m is an integer of 1 to 10, preferably 2.

A production process for the first and second peptide-lipid conjugate in the present invention is not particularly limited. For example, the first peptide-lipid conjugate represented by the general formula 2 can be produced by allowing a long-chain carboxylic acid represented by the following general formula: R²-COOH (wherein R² represents the same as R² in the general formula 2) or a long-chain carboxylic acid chloride represented by the following general formula: R²-COCl (wherein R² represents the same as R² in the general formula 2) to react with an N-terminal region of a peptide so as to form a peptide bond.

The long-chain carboxylic acid may include hexanic acid, heptanic acid, octanic acid, nonanic acid, decanic acid, undecanic acid, dodecanic acid, tridecanic acid, tetradecanic acid, pentadecanic acid, hexadecanic acid, heptadecanic acid, octadecanic acid, nonadecanic acid, eicosanic acid, heneicosanic acid, docosanic acid, tricosanic acid, tetracosanic acid, pentacosanic acid and hexacosanic acid. Among them, dodecanic acid and tetradecanic acid are preferable in view of their advantage of good balance of amphiphilicity provided to the resulting peptide-lipid conjugate, and low-cost high availability because they occur in nature.

The second peptide-lipid conjugate represented by the general formula 3 can be produced by allowing a long-chain amine represented by the following general formula: R²-NH₂ (wherein R² represents the same meaning as R² in the general formula 3) to react with a C-terminal region of a peptide so as to form a peptide bond.

The long-chain amine may include hexyl amine, heptyl amine, octyl amine, nonyl amine, decyl amine, undecyl amine, dodecyl amine, tridecyl amine, tetradecyl amine, pentadecyl amine, hexadecyl amine, heptadecyl amine, octadecyl amine, nonadecyl amine, eicosyl amine, heneicosyl amine, docosyl amine, tricosyl amine, tetracosyl amine, pentacosyl amine and hexacosyl amine. Among them, tetradecyl amine and hexadecyl amine are preferable in view of their advantage of good balance of amphiphilicity provided to the resulting peptide-lipid conjugate, and low-cost high availability.

Generally, an N-terminal of the second peptide-lipid conjugate represented by the general formula 3 is isolated as a salt of an acid having a halogen atom. This is performed to deprotect the N-terminal modified by various protective groups, using various dilute acids/organic solvents. Further, as compared with keeping an N-terminal amino group in a free state, it is more chemically stable to keep the N-terminal amino group as a salt of an acid having a halogen atom. The salt is generally a hydrobromide or a hydrochloride, typically a hydrochloride salt.

SYNTHESIS OF WATER-FREE ORGANIC NANOTUBE

A method of producing a water-free organic nanotube using the N-glycoside type glycolipid or the first or second peptide-lipid conjugate will be described.

[1a] Firstly, the N-glycoside type glycolipid or the first or second peptide-lipid conjugate is dissolved in an organic solvent to prepare a solution. A distinctive feature is to use an organic solvent instead of water. This organic solvent is heated up to a temperature equal to or less than a boiling point thereof. This makes it possible to increase the amount of N-glycoside type glycolipid dissolved therein. A higher concentration of the N-glycoside type glycolipid or the first or second peptide-lipid conjugate in the solution is preferable, a saturated state is most preferable. The organic solvent to be used may be an alcohol-based solvent or a cyclic ether-based solvent, each having a boiling point of 120°C or less. A single organic solvent or a mixture of two or more types of solvents may be a single type of solvent or may be used.

Further, one or more selected from the group consisting of an aromatic hydrocarbon-based solvent, a paraffin-based solvent, a paraffin chloride-based solvent, an olefin chloride-based solvent, an aromatic hydrocarbon chloride-based solvent, a straight-chain ether-based solvent, a ketone-based solvent, an ester-based solvent and a nitrogen-containing compound-based solvent may be mixed to the alcohol-based solvent or the cyclic ether-based solvent. This mixed solvent contains the alcohol-based solvent or the cyclic ether-based solvent preferably in an amount of at least 30 volume%, more preferably in an amount of at least 50 volume%.

The alcohol-based solvent may include 1-butanol, 2-butanol, ethanol, methanol, 2-methoxyethanol, isobutyl alcohol, tertiary butyl alcohol, 2-pentanol, 1-propanol, 2-propanol, amyl alcohol and allyl alcohol.

The cyclic ether-based solvent may include dioxane and tetrahydrofuran.

The aromatic hydrocarbon-based solvent may include benzene, toluene, mesitylene, tetralin (tetrahydronaphthalene) and xylene.

The paraffin-based solvent may include cyclohexane, heptane, hexane, ligroin, pentane, ethyl cyclopentane, petroleum ether, isooctane, isohexane, isoheptane, isopentane, decalin (decahydronaphthalene), decane, dodecane, octane and nonane.

The paraffin chloride-based solvent may include carbon tetrachloride, chloroform, 1,1-dichloroethane, 1,2-dichloroethane, methylene chloride, ethyl chloride, butyl chloride and propyl chloride.

The olefin chloride-based solvent may include tetrachloroethylene, trichloroethylene, 1,1-dichloroethylene and 1,2-dichloroethylene.

The aromatic hydrocarbon chloride-based solvent may include 1-chloronaphthalene, o-dichlorobenzene and m-dichlorobenzene.

The straight-chain ether-based solvent may include diethyl ether, diisopropyl ether, 1,2-dimethoxymethane, dimethoxymethane, ethyl methyl ether, dibenzyl ether, diphenyl ether and triglyme.

The ketone-based solvent may include acetone, methyl ethyl ketone, acetal, acetaldehyde, cyclohexane, methyl isobutyl ketone and dimethylsulfoxide.

The ester-based solvent may include ethyl acetate, ethyl formate, methyl acetate, methyl formate, butyl formate, propyl formate, isopropyl formate, propyl acetate, ethyl propionate, methyl propionate, ethyl butyrate and methyl butyrate.

The nitrogen-containing compound-based solvent may include acetamide, acetonitrile, diethylamine, di-isopropylamine, ethylamine, ethylenediamine, hydrazine, nitromethane, piperidine, propylamine, pyridine, N,N,N',N'-tetramethylethylene diamine, triethylamine, acrylonitrile, aniline, di-isopropyethylamine, dimethylacetamide, dimethylaniline, N,N-dimethylformamide, formamide, N-methylpyrrolidone, morpholine, nitrobenzene and quinoline.

The N-glycoside type glycolipid solution or first or second peptide-lipid conjugate solution prepared in the above manner is slowly cooled, and then maintained undisturbed under room temperature to form water-free organic nanotubes. The term "slow cooling" has two meanings: one is to allow a temperature fall without having any heating and cooling operation; the other is to allow a temperature fall under control based on a heating and cooling operation. Thus, there are two cases: one case where the temperature during the slow cooling varies depending on an ambient temperature and a heat capacity of devices; the other case where the temperature during the slow cooling is dependent on a setting of a device for controlling the temperature. The term "room temperature" means that a temperature is not subject to particularly excessive heating or cooling. Specifically, the room temperature means a temperature of 0 to 40°C, preferably around 20°C. After an elapse of a slow cooling period often-odd hours to several days, a hollow fibrous substance is precipitated from the solution.

[1b] As an alternative method, the N-glycoside type glycolipid or the first or second peptide-lipid conjugate is dissolved in the same organic solvent as described above to prepare a solution. In this method, it is not particularly necessary to heat the solvent before use. A concentration of the N-glycoside type glycolipid or the first or second peptide-lipid conjugate in the solution is preferably increased as high as possible, most preferably brought to a saturated state.

Then, the solution is concentrated. For example, the solution is concentrated and dried by using an evaporator, preferably at an evaporation temperature ranging from room temperature to its boiling temperature in a low vacuum, under a pressure of 5 to 10 KPa.

As a result, water-free organic nanotubes are precipitated from the solution depending on a solubility to the organic solvent.

[1c] As another alternative method, the N-glycoside type glycolipid or the first or second peptide-lipid conjugate is dissolved in the same organic solvent as described above to prepare a solution. In this method, it is not particularly necessary to heat the solvent before use. A higher concentration of the N-glycoside type glycolipid or the first or second peptide-lipid conjugate in the solution is preferable. A saturated state in the solution is most preferable.

Then, a poor solvent for the N-glycoside type glycolipid or the first or second peptide-lipid conjugate is added to the solution, preferably in an amount of at least 100 volume%, more preferably in an amount of at least 300 volume%, with respect to the already added organic solvent.

The poor solvent to be used may be any one of an aromatic hydrocarbon-based solvent, a paraffin-based solvent, a paraffin chloride-based solvent, an olefin chloride-based solvent, an aromatic hydrocarbon chloride-based solvent, a straight-chain ether-based solvent, a ketone-based solvent, an ester-based solvent and a nitrogen-containing compound-based solvent. The poor solvent may also be a mixture of two or more of the above organic solvents.

The cyclic ether-based solvent may include dioxane and tetrahydrofuran.

The aromatic hydrocarbon-based solvent may include benzene, toluene, mesitylene, tetralin (tetrahydronaphthalene) and xylene.

The paraffin-based solvent may include cyclohexane, heptane, hexane, ligroin, pentane, ethyl cyclopentane, petroleum ether, isooctane, isohexane, isoheptane, isopentane, decalin (decahydronaphthalene), decane, dodecane, octane and nonane.

The paraffin chloride-based solvent may include carbon tetrachloride, chloroform, 1,1-dichloroethane, 1,2-dichloroethane, methylene chloride, ethyl chloride, butyl chloride and propyl chloride.

The olefin chloride-based solvent may include tetrachloroethylene, trichloroethylene, 1,1-dichloroethylene and 1,2-dichloroethylene.

The aromatic hydrocarbon chloride-based solvent may include 1-chloronaphthalene, o-dichlorobenzene and m-dichlorobenzene.

The straight-chain ether-based solvent may include diethyl ether, diisopropyl ether, 1,2-dimethoxymethane, dimethoxymethane, ethyl methyl ether, dibenzyl ether, diphenyl ether and triglyme.

The ketone-based solvent may include acetone, methyl ethyl ketone, acetal, acetaldehyde, cyclohexane, methyl isobutyl ketone and dimethylsulfoxide.

The ester-based solvent may include ethyl acetate, ethyl formate, methyl acetate, methyl formate, butyl formate, propyl formate, isopropyl formate, propyl acetate, ethyl propionate, methyl propionate, ethyl butyrate and methyl butyrate.

The nitrogen-containing compound-based solvent may include acetamide, acetonitrile, diethylamine, di-isopropylamine, ethylamme, ethylenediamine, hydrazine, nitromethane, piperidine, propylamine, pyridine, N,N,N,N'-tetramethylethylene diamine, triethylamine, acrylonitrile, aniline, di-isopropyethylamine, dimethylacetamide, dimethylaniline, N,N-dimethylformamide, formamide, N-methylpyrrolidone, morpholine, nitrobenzene and quinoline.

As a result, water-free organic nanotubes are precipitated from the solution depending on a solubility to the organic solvent.

The water-free organic nanotubes obtained through the synthesis method [1a] are characterized in that each of them has a high axial ratio, and they have an entangled structure. The water-free organic nanotubes obtained through the synthesis method [1b] are characterized in that each of them has a low axial ratio, and they are obtained as relatively large aggregate. The water-free organic nanotubes obtained through the synthesis method [1c] are characterized in that each of them has a low axial ratio, and they are obtained in a dispersed state.

[2] Then, the water-free organic nanotubes are collected from the solution, and dried in air or by heating under reduced pressure. The obtained water-free organic nanotubes has an average outer diameter of 70 to 500 nm, preferably 200 to 500 nm, an average inner diameter (an average diameter of a hollow space) of 40 to 300 nm, preferably 50 to 300 nm, and a length of several hundred nm to several hundred µm, and exhibits stability in air.

The "drying in air (air-drying)" means that the collected water-free organic nanotubes are dried in a space having airflow at room temperature under atmospheric pressure. The drying time is in the range of 2 to 48 hours, preferably 24 hours or more.

The "drying by heating under reduced pressure" means that the collected water-free organic nanotubes are dried at a temperature ranging from room temperature to 60°C in a vacuum of 20 Pa or below for 2 hours or more. Although a drying time can be reduced by increasing a heating temperature to 60°C or more, the heating temperature is preferably set at 50°C or less in consideration of an influence on a thermal stability of the lipid. More preferably, the drying is performed at a heating temperature of 30°C for 24 to 48 hours.

The water-free organic nanotubes obtained in the above manner contain no water inside each tube, and can be easily produced without containing any organic solvent therein, choosing manufacturing conditions.

A configuration and dimensions of the hollow fibrous organic nanotubes are checked using an optical microscope, a laser microscope, a scanning electron microscope, a transmission electron microscope, and/or an atomic force microscope. Generally, the optical microscope or the laser microscope has difficulty in detecting a fibrous structure having an outer diameter of several hundred nm or less, without using a particular technique, such as a staining technique. The scanning electron microscope is a significant by effective means for observation of a surface and configuration of a fibrous structure. Although a hollow cylinder structure can also be directly checked by this microscope, it is not always effective, depending on orientation of the fibrous structure. The transmission electron microscope can be used for checking a hollow cylinder structure because it is capable of expressing the hollow cylinder structure as a difference of contrasting density. However, it provides the same contrast image even if opposing lateral edges of a ribbon-like structure slightly curl up, and thereby it is somewhat risky to determine a hollow cylinder structure using only the transmission electron microscope. Thus, it is desirable to use the scanning electron microscope and the transmission electron microscope in combination in order to check a presence of a hollow fibrous configuration.

A process of encapsulating a functional substance into the water-free organic nanotubes obtained in the above manner will be described below.

Although a functional substance may be encapsulated into the water-free organic nanotubes through any process, it is preferable to perform the encapsulating through the following process. A desired functional substance can be introduced into the water-free organic nanotubes through a process comprising a step of drying the water-free organic nanotubes (first step), a step of dissolving or dispersing the functional substance in a solvent (second step), and a step of dispersing the freeze-dried water-free organic nanotubes over the solution or dispersion liquid at a temperature equal to or less than a gel-liquid phase transition temperature of the N-glycoside type glycolipid or the first or second peptide-lipid conjugate (third step).

The above process will be more specifically described on a step-by-step basis.

[3] First Step

In the first step, the water-free organic nanotubes are dried.

A temperature of the drying is set at room temperature, preferably 20°C or less. A pressure (vacuum) of the drying is preferably set at 50 Pa or less, more preferably 10 Pa or less. A time period of the drying is preferably set at 5 hours or more, more preferably 24 hours or more.

[4] Second Step

In the second step, a desired functional substance is dissolved or dispersed in a solvent. The solvent is water or an organic solvent, and a type of the solvent and a concentration of the functional substance may be appropriately selected depending on a type of the functional substance and an intended purpose.

The functional substance is not particularly limited, but may be appropriately selected depending on an intended purpose. If the functional substance is dissoluble in the solvent, a solution will be formed. If the functional substance is nanoparticles (preferably having a particle size of an atomic size to 50 nm) dispersable in the solvent although it is not dissoluble in the solvent, a dispersion liquid will be formed.

An example of the functional substance and the intended purpose thereof will be described below.

(a) Metal

In this case, metal may be prepared in the form of a metal salt to prepare an aqueous solution. The metal salt may include chloride salt, nitrate salt, sulfate salt, acetate salt and hydroxide salt.

(b) Nanoparticles of Metal, Metal Oxide or Metal Sulfide, Dendrimer, Carbon Nanotube, Polymer, Cyclodextrin

In this case, a colloid solution of this functional substance may be prepared.

(c) Physiological Active Substance

This functional substance can be directly used in the form of a solution. The physiological active substance may include immune protein, nucleic acid, gene, low-molecular organic compound, nonimmune protein, immunoglobulin binding protein, sugar binding protein, enzyme, microorganism and virus.

(d) Chemicals

This functional substance can be used in the form of a solution or dispersion liquid. The chemicals may include medical substances, such as a therapeutic agent, a diagnostic product and a contrast medium, and chemicals as active substances for cosmetics.

[5] Third Step

In the third step, the water-free organic nanotubes dried in the first step is dispersed over the solution or dispersion liquid of the functional substance prepared in the second step. It is believed that the solution or dispersion liquid of the functional substance is sucked inside the tubes by a suction force based on a capillary phenomenon. An amount of the water-free organic nanotubes to be added is not particularly limited, but may be appropriately selected depending on an intended final purpose, a concentration of the functional substance in the solution or dispersion liquid, and a size of each of the water-free organic nanotubes.

In this step, a temperature of the solvent is set to be equal to or less than a gel-liquid phase transition temperature of the N-glycoside type glycolipid or the first or second peptide-lipid conjugate, preferably normal or room temperature, i.e., a temperature causing no particular increase and decrease in temperature of the solvent.

The gel-liquid phase transition temperature can be measured by a differential scanning calorimetric analysis. Specifically, a sample prepared by mixing 1 to 5 mg of the N-glycoside type glycolipid or the first or second peptide-lipid conjugate with 30 to 50 µL of water in such a manner as to fully hydrate the compounds is subjected to a differential scanning calorimeter. Thus, a gel-liquid phase transition phenomenon is indicated as a heat-absorption peak, and a temperature at a position of the maximum peak is determined as the gel-liquid phase transition temperature.

The gel-liquid phase transition temperature means a melting point of a surfactant in water, in the colloid chemistry. If an aqueous dispersion is heated up to this temperature or more, a structure of the tube is quickly changed to a spherical vesicle configuration, and undesirably destroyed. The gel-liquid phase transition temperature is dependent on a type of a surface-active organic compound, and typically in the range of 30 to 90°C.

The dispersion is preferably performed under atmospheric pressure. Alternatively, the dispersion may be performed while applying a pressure of 0.2 MPa or less.

EXAMPLE

The present invention will be more specifically described based on examples. It is understood that such examples are not intended to limit the present invention.

[Synthesis Example 1: Synthesis of β-D-Glucopyranosylamine]

D-(+)-glucopyranose (produced by Fluka Chemie AG, 1.0 g, 5.55 mM) was taken in a flask, and 50 mL of water was added thereto to dissolve the D-(+)-glucopyranose therein. 10 g of ammonium hydrogencarbonate (produced by Wako Pure Chemical Industries, Ltd.) was added until a crystal was precipitated on a bottom of the flask. The solution was magnetically stirred in an oil bath at 37°C, for 3 to 5 days. During reaction, ammonium hydrogencarbonate was added on an intermittent basis to maintain a saturated state. A total amount of the added ammonium hydrogencarbonate was 40 to 50 g. The reaction was tracked by thin-layer chromatography (Rf value = 0.40, developing solvent: volume ratio of acetate ester/acetic acid/methanol/water (volume ratio: 4/3/3/1)).

As an aftertreatment, in order to remove an unreacted ammonium hydrogencarbonate from the reaction system, the solution was cooled to precipitate the unreacted ammonium hydrogencarbonate as a crystal. As an alternative technique, after adding an appropriate amount of water to the reaction system, the solution may be concentrated to evaporate the unreacted ammonium hydrogencarbonate. Further, a desalination apparatus may be used for removing the unreacted ammonium hydrogencarbonate. In this manner, β-D-glucopyranosyl amine was obtained.

[Example 1]

(Synthesis of N-(9-cis-octadecenoyl)-β-D-glucopyranosylamine water-free organic nanotubes)

Oleoyl chloride (produced by Aldrich Chemical Company, Inc., 514 µL, 1.1 mM) was added to an ethanol solution (10 mL) of the β-D-glucopyranosylamine (40 mg, 0.22 mM) obtained in the Synthesis Example 1 and triethylamine (111 mg, 1.1 mM), and the mixture was stirred at 0°C for 19 hours. A resulting crude product was subjected to silica gel column chromatography (elution solvent: chloroform/methanol = 4/1) to obtain N-(9-cis-octadecenoyl)-β-D-glucopyranosylamine (24 mg, yield: 25%) in the form of a white solid substance.

| | |
|---|---|
| Melting point: | 149°C |
| Elemental analysis: | C₂₄H₄₅O₆N |
| Calculated value (%): | C64.98, H10.22, N3.16 |
| Actual measurement value (%): | C63.05, H9.85, N2.93 |

2 g of the obtained N-(9-cis-octadecenoyl)-β-D-glucopyranosylamine was added to 30 mL of a mixed solvent of ethyl acetate/methanol (volume ratio: 5/1). The mixture was heated at 60 °C for 10 minutes to dissolve the N-(9-cis-octadecenoyl)- β-D-glucopyranosylamine in the solvent. Then, the solution was naturally cooled down to 20°C and maintained in a stationary state to obtain N-(9-cis-octadecenoyl)-β-D-glucopyranosylamine water-free organic nanotubes (weight: 1.2 g).

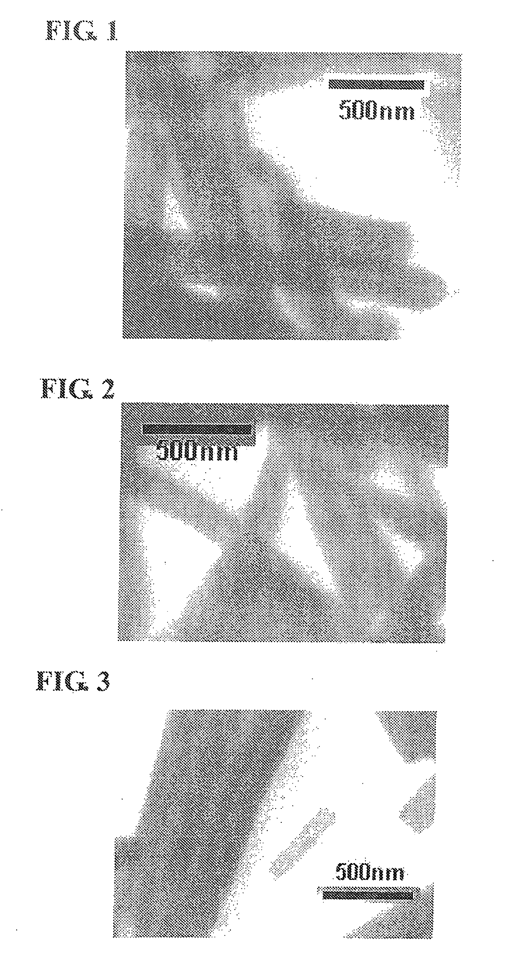

FIG 1 shows a scanning transmission electron microscopic (STEM) observation image of the obtained water-free organic nanotubes.

[Example 2]

(Synthesis of N-(11-cis-ocβtadecenoyl)-β-D-glucopyranosylamine water-free organic nanotubes)

The β-D-glucopyranosylamine (1.24 g, 6.9 mM) obtained in the Synthesis Example 1 was added to a dimethylsulfoxide solution (2.5 mL) of 11-cis-octadecenoic acid (282 mg, 1.0 mM), HOBt (153 mg, 1.0 mM) and BOP (1.33 g, 3.0 mM), and the mixture was stirred at room temperature for 45 hours. A resulting crude product was subjected to silica gel column chromatography (elution solvent: chloroform/methanol = 4/1) to obtain N-(11-cis-octadecenoyl)- β-D-glucopyranosylamine (85 mg, yield: 19%) in the form of a white solid substance.

| | |
|---|---|
| Melting point: | 148°C |
| Elemental analysis: | C₂₄H₄₅O₆N |
| Calculated value (%): | C64.98, H10.22, N3.16 |
| Actual measurement value (%): | C63.68, H10.02, N3.16 |

The obtained N-(11-cis-octadecenoyl)-β-D-glucopyranosylamine (weight: 14 g) was added to 200 mL of a mixed solvent of ethyl acetate/methanol (volume ratio: 3/1). The mixture was heated at 60°C for 10 minutes to dissolve the N-(11-cis-octadecenoyl)-β-D-glucopyranosylamine in the solvent. Then, the solution was naturally cooled down to 20°C and maintained in a stationary state to obtain N-(11-cis-octadecenoyl)-β-D-glucopyranosylamine water-free organic nanotubes (weight: 8.7 g).

FIG 2 shows a scanning transmission electron microscopic (STEM) observation image of the obtained water-free organic nanotubes.

| | |
|---|---|
| Elemental analysis of water-free organic nanotubes: | C₂₄E₄₅O₆N |
| Calculated value (%): | C64.98, H10.22, N3.16, 021.64 |
| Actual measurement value (%): | C63.68, H10.02, N3.16, 023.14 |

[Example 3]

(Synthesis of N-(13-cis-octadecenoyl)-β-D-glucopyranosylamine water-free organic nanotubes)

The β-D-glucopyranosylamine (877 mg, 4.9 mM) obtained in the Synthesis Example 1 was added to a dimethylsulfoxide solution (2.5 mL) of 13-cis-octadecenoic acid (350 mg, 1.24 mM), HOBt (190 mg, 1.24 mM) and BOP (1.65 g, 3.72 mM), and the mixture was stirred at room temperature for 45 hours. A resulting crude product was subjected to silica gel column chromatography (elution solvent: chloroform/methanol = 4/1) to obtain N-(13-cis-octadecenoyl)- β-D-glucopyranosylamine (166 mg, yield: 30%) in the form of a white solid substance.

| | |
|---|---|
| Melting point: | 148°C |
| Elemental analysis: | C₂₄H₄₅O₆N |
| Calculated value (%): | C64.98, H10.22, N3.16 |
| Actual measurement value (%): | C63.82, H9.97, N3.17 |

166 mg of the obtained N-(13-cis-octadecenoyl)-β-D-glucopyranosylamine was dissolved in 42 mL of methanol. The solution was concentrated and dried using a rotary evaporator (pressure: 10 KPa, evaporation temperature: 40°C), and a resulting residue was further dried in vacuum (10 Pa) at 60°C for 3 hours to obtain N-(13-cis-octadecenoyl)-β-D-glucopyranosylamine water-free organic nanotubes (yielding: 160 mg).

FIG 3 shows a scanning transmission electron microscopic (STEM) observation image of the obtained water-free organic nanotubes.

[Example 4]

(Synthesis of N-tetradecanoyl-glycyl-glycine water-free organic nanotubes)

0.31 mL (2.2 mM) of triethylamine was added to 0.57 g (2.2 mM) of glycyl-glycine benzyl ester hydrochloride, and the mixture was dissolved in 10 mL of ethanol. Then, 50 ml of a chloroform solution containing 0.46 g (2 mM) of tridecanecarboxylic acid was added thereto. Then, 20 mL of a chloroform solution containing 0.42 g (2.2 mM) of 1-ethyl-3-(3-dimethylaminopropyl) carbodiimide hydrochloride was added to the mixed solution under cooling at - 10°C, and the obtained solution was stirred for one day while gradually retuning a temperature thereof to room temperature. A resulting reacted solution was rinsed by 50 mL of an aqueous solution of citric acid (10 weight%), 50 mL of an aqueous solution of sodium hydrogencarbonate (4 weight%) and 50 mL of purified water, and then concentrated and dried using a rotary evaporator (pressure: 10 KPa, evaporation temperature: 40°C) to obtain 0.57 g (yield: 65%) of N-tetradecanoyl-glycyl-glycine benzyl ester. 0.43g (1 mM) of the obtained compound was dissolved in 100 mL of dimethylformamide (DMF), and then subjected to catalytic hydrogen reduction in the presence of 0.5 g of 10 weight% palladium/carbon as a catalyst. After an elapse of 6 hours, a resulting solution was filtered through a sellite filter, and then concentrated and dried using a rotary evaporator (pressure: 10 KPa, evaporation temperature: 40°C) to obtain 0.21 g (yield: 60%) of N-tetradecanoyl-glycyl-glycine which is represented by the general formula 2: R²CO (NH-CHR³-CO)ₘ OH, wherein: R² is a tridecyl group; m is 2; and each R³ is H.

| | |
|---|---|
| Melting point: | 158°C |
| Elemental analysis: | C₁₈H₃₄N₂O₄ |
| Calculated value (%): | C63.13, H10.01, N8.18 |
| Actual measurement value (%): | C62.09, H9.65, N8.25 |

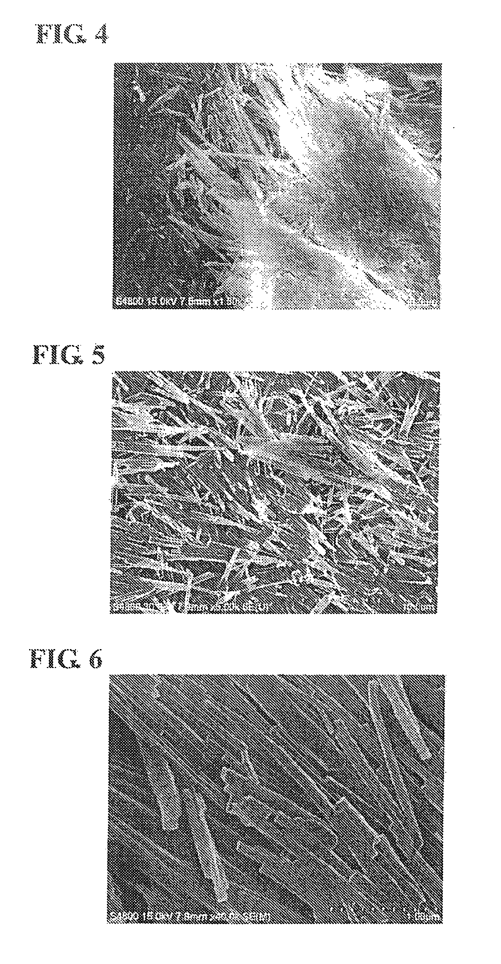

0.5 g of the obtained N-tetradecanoyl-glycyl-glycine was dissolved in 50 mL of methanol which was being heated up to 40°C. Then, the solution was concentrated and dried using a rotary evaporator (pressure: 10 KPa, evaporation temperature: 40°C) to precipitate a white powder. The obtained white powder was further air-dried for 24 hours to obtain N-tetradecanoyl-glycyl-glycine water-free organic nanotubes (yielding: 0.48 g). FIG. 4 shows a scanning electron microscopic (SEM) observation image of the water-free organic nanotubes. Through observation using a transmission electron microscope and a scanning electron microscope, it was verified that hollow fibrous organic nanotubes having an average outer diameter of 200 nm are formed.

[Example 5]

0.5 g of the N-tetradecanoyl-glycyl-glycine obtained in the Example 4 was dissolved in 50 mL of ethanol which was being heated up to 40°C. Then, the solution was concentrated and dried using a rotary evaporator (pressure: 10 KPa, evaporation temperature: 40°C) to precipitate a white powder. The obtained white powder was further air-dried for 48 hours to obtain N-tetradecanoyl-glycyl-glycine water-free organic nanotubes (yielding: 0.46 g). FIG 5 shows a scanning electron microscopic (SEM) observation image of the obtained water-free organic nanotubes. Through observation using a transmission electron microscope and a scanning electron microscope, it was verified that water-free organic nanotubes having an average outer diameter of 220 nm are formed.

[Example 6]

0.5 g of the N-tetradecanoyl-gycyl-glycine obtained in the Example 4 was dissolved in a mixed solvent of methanol (25 mL) and chloroform (25 mL) which was being heated up to 40°C. Then, the solution was concentrated and dried using a rotary evaporator (pressure: 10 KPa, evaporation temperature: 40°C) to precipitate a white powder. The obtained white powder was further air-dried for 24 hours to obtain N-tetradecanoyl-glycyl-glycine water-free organic nanotubes (yielding: 0.48 g). FIG. 6 shows a scanning electron microscopic (SEM) observation image of the obtained water-free organic nanotubes. Through observation using a transmission electron microscope and a scanning electron microscope, it was verified that water-free organic nanotubes having an average outer diameter of 80 nm are formed.

[Example 7]

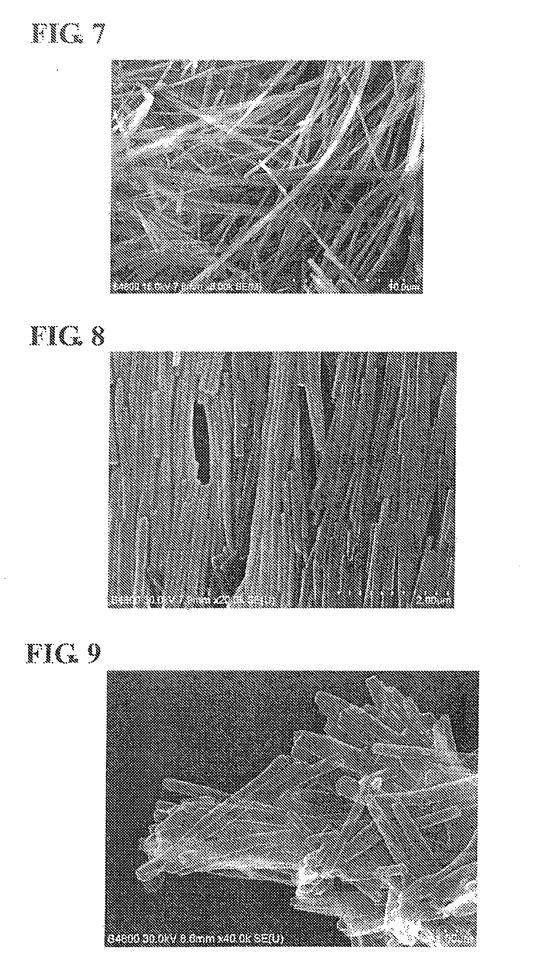

0.5 g of the N-tetradecanoyl-glycyl-glycine obtained in the Example 4 was dissolved in a mixed solvent of ethanol (25 mL) and chloroform (25 mL), being heated up to 40°C. Then, the solution was concentrated and dried using a rotary evaporator (pressure: 10 KPa, evaporation temperature: 40°C) to precipitate a white powder. The obtained white powder was further dried by heating at 40°C under reduced pressure (10 Pa) for 3 hours to obtain N-tetradecanoyl- glycyl-glycine water-free organic nanotubes (yielding: 0.45 g). FIG 7 shows a scanning electron microscopic (SEM) observation image of the obtained water-free organic nanotubes. Through observation using a transmission electron microscope and a scanning electron microscope, it was verified that water-free organic nanotubes having an average outer diameter of 140 nm are formed.

[Example 8]

0.5 g of the N-tetradecanoyl-glycyl-glycine obtained in the Example 4 was dissolved in a mixed solvent of DMF (40 mL), ethanol (9 mL) and water (1 mL) heated up to 40°C. Then, the solution was concentrated and dried using a rotary evaporator (pressure: 10 KPa, evaporation temperature: 60°C) to precipitate a white powder. The obtained white powder was further dried by heating at 60°C under reduced pressure (10 Pa) for 3 hours to obtain N-tetradecanoyl- glycyl-glycine water-free organic nanotubes (yielding: 0.48 g). FIG 8 shows a scanning electron microscopic (SEM) observation image of the obtained water-free organic nanotubes. Through observation using a transmission electron microscope and a scanning electron microscope, it was verified that water-free organic nanotubes having an average outer diameter of 125 nm are formed.

[Example 9]

(Synthesis of N-tetradecyl-glycyl-glycine amide water-free organic nanotubes)

0.3I mL (2.2 mM) of triethylamine was added to 0.51 g (2.2 mM) of t-butyloxycarbonyl- glycyl-glycine, and the mixture was dissolved in 10 mL of ethanol. 50 ml of a chloroform solution containing 0.43 g (2 mM) of tetradecylamine was added thereto. Then, 20 mL of a chloroform solution containing 0.42 g (2.2 mM) of 1-ethyl-3-(3-dimethylaminopropyl) carbodiimide hydrochloride was added to the mixed solution under cooling at - 10°C, and the obtained solution was stirred for one day while gradually retuning a temperature thereof to room temperature. A resulting reacted solution was rinsed by 50 mL of an aqueous solution of citric acid (10 weight%), 50 mL of an aqueous solution of sodium hydrogencarbonate (4 weight%) and 50 mL of purified water, and then concentrated and dried using a rotary evaporator (pressure: 10 KPa, evaporation temperature: 40°C) to obtain oil (N-tetradecyl-t-butyloxycarbonyl- glycyl-glycine amide). The obtained oil was dissolved in 100 mL of chloroform, and 4N hydrochloric acid/ethyl acetate was added thereto to deprotect peptide. After an elapse of 4 hours, the solution was concentrated and dried using a rotary evaporator (pressure: 10 KPa, evaporation temperature: 40°C) to obtain 0.38 g (yield: 52%) of N-tetradecyl-glycyl-glycine amide hydrochloride.

| | |
|---|---|
| Melting point: | 150°C |
| Elemental analysis: | C₁₈H₃₈ClN₃O₂ · H₂O |
| Calculated value (%): | C56.60, H10.56, N11.00 |
| Actual measurement value (%): | C56.10, H10.88, N11.44 |

The obtained N-tetradecyl-glycyl-glycine amide (0.18 g) was dissolved in triethylamine (7 mL) and ethanol (100 mL), which were being heated up to 35°C. Then, the solvent was removed to obtain N-tetradecyl-glycyl-glycine amide water-free organic nanotubes (yielding: 0.16 g). FIG 9 shows a scanning electron microscopic (SEM) observation image of the obtained water-free organic nanotubes.

[Example 10]

(Synthesis of N-(glycyl-glycine) pentadecyl amide water-free organic nanotubes)

0.31 mL (2.2 nine) of triethylamine was added to 0.51 g (2.2 mM) of t-butyloxycarbonyl- glycyl-glycine, and the mixture was dissolved in 10 mL of ethanol. 50 ml of a chloroform solution containing 0.46 g (2 mM) of pentadecylamine was added thereto. Then, 20 mL of a chloroform solution containing 0.42 g (2.2 mM) of 1-ethyl-3-(3-dimethylaminopropyl) carbodiimide hydrochloride was added to the mixed solution under cooling at - 10°C, and the obtained solution was stirred for one day while gradually returning a temperature thereof to room temperature. A resulting reacted solution was rinsed by 50 mL of an aqueous solution of citric acid (10 weight%), 50 mL of an aqueous solution of sodium hydrogencarbonate (4 weight%) and 50 mL of purified water, and then concentrated under reduced pressure to obtain oil (N-(t-butyloxycarbonyl-glycyl-glycine) pentadecyl amide). The obtained oil was dissolved in 100 mL of chloroform, and 10 mL of 4N hydrochloric acid/ethyl acetate was added thereto to deprotect peptide. After an elapse of 4 hours, the solution was concentrated under reduced pressure to obtain 0.59 g (yield: 78%) of N-(t-butyloxycarbonyl-glycyl-glycine) pentadecyl amide hydrochloride.

| | |
|---|---|
| Melting point: | 135°C |
| Elemental analysis: | C₁₉H₄₀ClN₃O₂ |
| Calculated value (%): | C60.37, H10.67, N11.12 |
| Actual measurement value (%): | C60.50, H10.31, N10.82 |

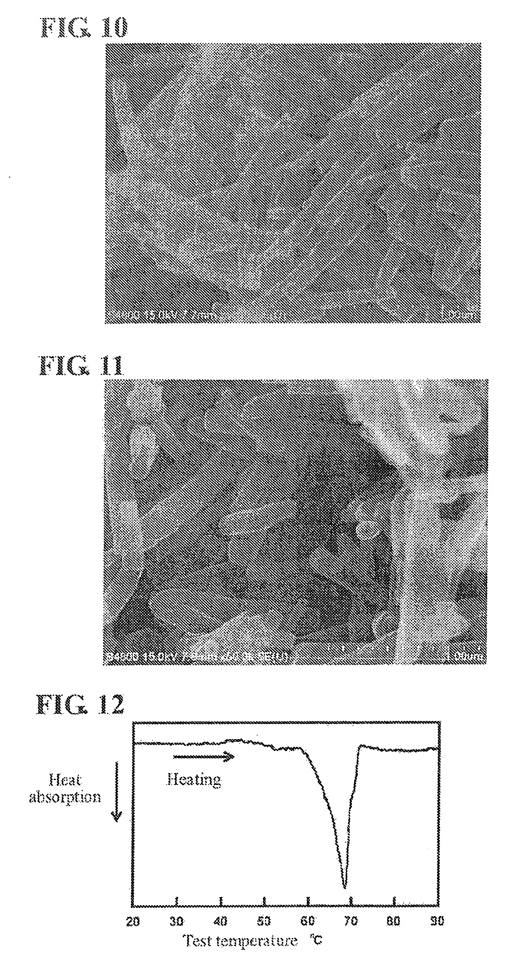

The obtained N-(t-butyloxycarbonyl-glycyl-glycine) pentadecyl amide (0.19 g) was dissolved in triethylamine (7 mL) and ethanol (100 mL) heated up to 35°C. Then, the solvent was removed to obtain N-(glycyl-glycine) pentadecyl amide water-free organic nanotubes (yielding: 0.19 g). FIG. 10 shows a scanning electron microscopic (SEM) observation image of the obtained water-free organic nanotubes. Through electron microscopic observation, it was verified that water-free organic nanotubes having an average outer diameter of 95 nm are formed.

[Example 11]

(Synthesis of N-(glycyl-glycine) hexadecyl amide water-free organic nanotubes)

0.31 mL (2.2 mM) of triethylamine was added to 0.51 g (2.2 mM) of t-butyloxycarbonyl- glycyl-glycine, and the mixture was dissolved in 10 mL of ethanol. 50 ml of a chloroform solution containing 0.48 g (2 mM) of hexadecylamine was added thereto. Then, 20 mL of a chloroform solution containing 0.42 g (2.2 mM) of 1-ethyl-3-(3-dimethylaminopropyl) carbodiimide hydrochloride was added to the mixed solution under cooling at - 10°C, and the obtained solution was stirred for one day while gradually retuning a temperature thereof to room temperature. A resulting reacted solution was rinsed by 50 mL of an aqueous solution of citric acid (10 weight%), 50 mL of an aqueous solution of sodium hydrogencarbonate (4 weight%) and 50 mL of purified water, and then concentrated under reduced pressure to obtain oil (N-(t-butyloxycarbonyl-glycyl-glycine) hexadecyl amide). The obtained oil was dissolved in 100 mL of chloroform, and 10 mL of 4N hydrochloric acid/ethyl acetate was added thereto to deprotect peptide. After an elapse of 4 hours, the solution was concentrated under reduced pressure to obtain 0.61 g (yield: 68%) of N-(t-butyloxycarbonyl-glycyl-glycine) hexadecyl amide hydrochloride.

| | |
|---|---|
| Melting point: | 145°C |
| Elemental analysis: | C₂₀H₄₂ClN₃O₂ |
| Calculated value (%): | C61.27, H10.80, N10.72 |
| Actual measurement value (%): | C61.55, H10.99, N10.50 |

The obtained N-(t-butyloxycarbonyl-glycyl-glycine) hexadecyl amide (0.20 g) was dissolved in triethylamine (7 mL) and ethanol (100 mL) heated up to 35°C. Then, the solvent was removed to obtain N-(glycyl-glycine) hexadecyl amide water-free organic nanotubes (yielding: 0.20 g). FIG. 11 shows a scanning electron microscopic (SEM) observation image of the water-free organic nanotubes. Through electron microscopic observation, it was verified that water-free organic nanotubes having an average outer diameter of 85 nm are formed.

[Comparative Example 1]

2 L of water was added to 100 mg of the N-(11-cis-octadecenoyl)-β-D-glucopyranosylamine obtained in the Example 2, and the solution was stirred for 30 minutes while being heated at 95°C. Then, the solution was maintained undisturbed for 15 hours to obtain a colloid dispersion having a fibrous structure. 2L of the obtained aqueous dispersion including water-containing organic nanotubes was subjected to centrifugal separation (3000 rpm) at room temperature for 10 minutes, and a supernatant liquid was fully removed by decantation. The water-containing organic nanotubes remaining at the bottom of a centrifuging tube was air-dried at room temperature for one hour. In the following Test Examples 1 and 2, the water-containing organic nanotubes prepared in the above manner were used.

In the following elemental analysis, a sample prepared by further freeze-drying (10 Pa) the water-containing organic nanotubes for 10 hours was used.

### Elemental analysis of water-containing organic nanotubes: C₂₄H₄₅O₆N · 2H₂O

| | |
|---|---|
| Calculated value (%): | C60.10, H10.30, N2.92, 026.68 |
| Actual measurement value (%): | C60.56, H9.82, N2.71, 026.92 |

As the result of elemental analusis on the obtained water-containing organic nanotubes, it was verified that two molecules of water are included per molecule of N-(11-cis-octadecenoyl) -β-D-glucopyranosylamine.

[Test Example 1]

The water-free organic nanotube obtained in the Example 2 and the water-containing organic nanotube obtained in the Comparative Example 1 were compared with each other in terms of a result of melting point measurement by differential scanning calorimetry.

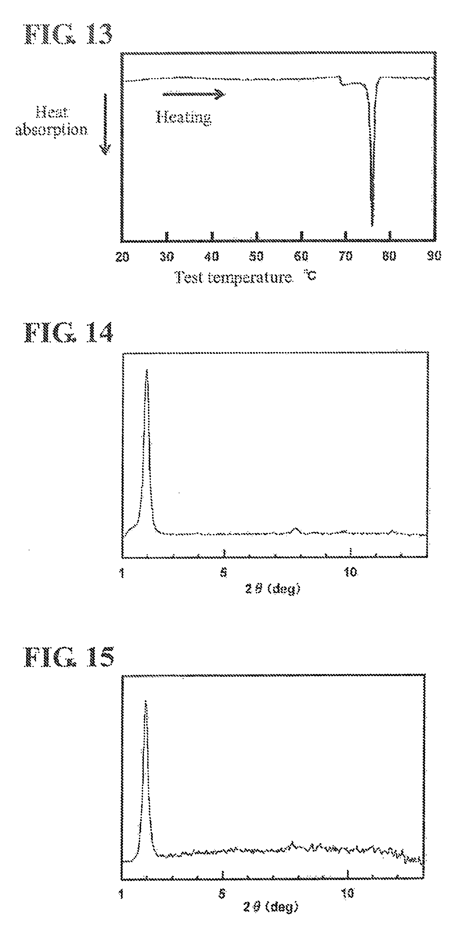

FIG 12 shows a differential scanning calorimetry curve of the water-free organic nanotube (Example 2). A melting point thereof was 68°C. FIG. 13 shows a differential scanning calorimetry curve of the water-containing organic nanotube (Comparative Example 1). A melting point thereof was 75°C.

In view of the fact that the melting point of the water-containing organic nanotube is greater than that of the water-free organic nanotube by 7°C, and the differential scanning calorimetry curve of the water-containing organic nanotube shows a sharper heat-absorption peak as compared with the differential scanning calorimetry curve of the water-free organic nanotube, it is proven that the water-containing organic nanotube has a bimolecular film structure with higher thermal stability and ordered configuration, based on water molecules included therein. This shows that water molecules of water-containing organic nanotube significantly solidly exist between layers of the bimolecular film structure.

Then, the two types of organic nanotubes were subjected to powder X-ray diff-ractometry to perform a comparative evaluation on an interplanar spacing.

FIG. 14 shows a powder X-ray diffraction pattern of the water-containing organic nanotube (Comparative Example 1). An interplanar spacing thereof was 4.53 nm. FIG 15 shows a powder X-ray diffraction pattern of the water-free organic nanotube (Example 2). An interplanar spacing thereof was 4.44 nm.

In view of the fact that the interplanar spacing of the water-containing organic nanotube is greater than that of the water-free organic nanotube, it is proven that the interplanar spacing of the water-containing organic nanotube becomes larger due to water molecules existing between the layers of the bimolecular film structure.

[Test Example 2]

The two types of organic nanotubes were subjected to time-series infrared spectrometry to perform a comparative evaluation on a temporal change in water amount.

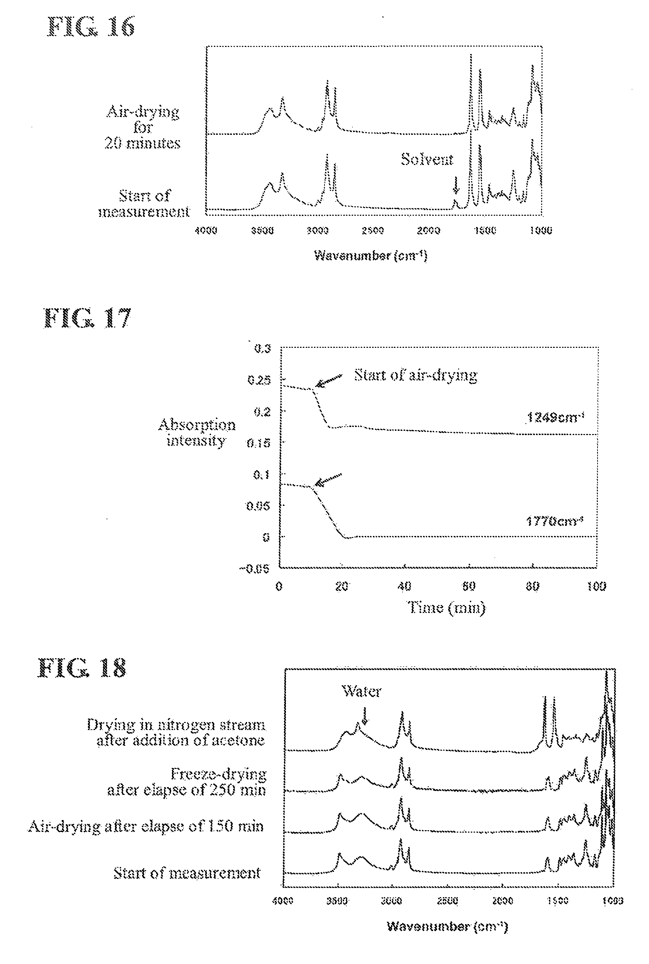

FIG 16 shows an infrared absorption spectrum of the water-free organic nanotube (Example 2) in a state after air-drying for 20 minutes. FIG 17 shows a temporal change in absorption intensity at each of 1249 cm⁻¹ and 1770 cm⁻¹ which are stretching vibration peaks of an ester group indicative of an amount of organic solvent (ethyl acetate) in the water-free organic nanotube.

Based on a result of this measurement, it was verified that the water-free organic nanotube synthesized using an organic solvent allows organic solvent components to be removed by drying at room temperature and normal pressure for one hour or less, in a significantly simple manner.

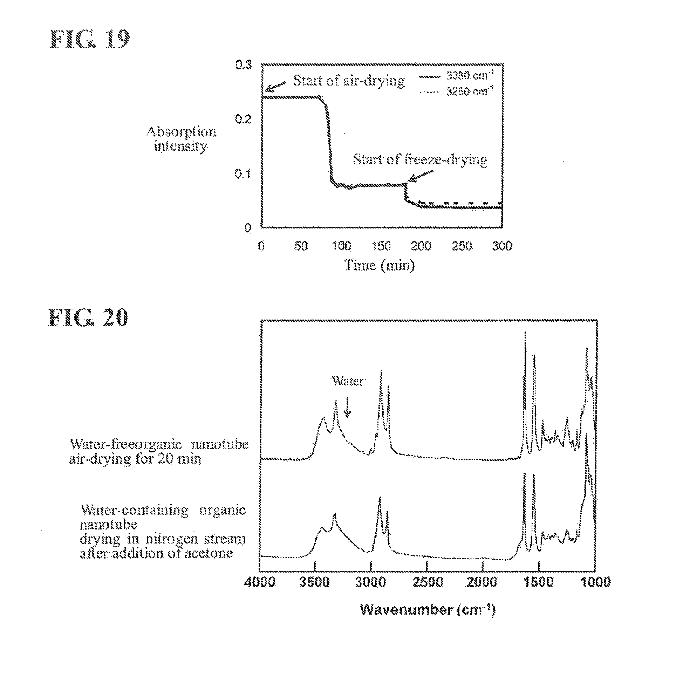

FIG. 18 shows time-series infrared absorption spectra of the water-containing organic nanotube (Comparative Example 1). Specifically, FIG. 18 shows respective time-series infrared absorption spectra of the water-containing organic nanotube (Comparative Example 1) in a state after it is air-dried for 150 minutes, in a state after it is freeze-dried for 75 minutes from completion of the air-drying for 175 minutes (i.e., in a state after an elapse of 250 minutes from start of the air-drying), and in a state after acetone is added thereto and then it is dried in a nitrogen stream for 60 minutes from completion of the freeze-drying for 125 minutes. FIG 19 shows a temporal change in absorption intensity with focus on each of 3380 cm⁻¹ and 3250 cm⁻¹ which are stretching vibration peaks of a hydroxyl group indicative of an amount of water in the water-containing organic nanotube. As seen in FIG. 19, it is difficult to fully remove water from the water-containing organic nanotube synthesized using water, even after an elapse of 300 minutes from start of air-drying, i.e., after freeze-drying in vacuum. As seen in FIG. 18, it is necessary to perform an additional operation, such as an operation of adding acetone to the water-containing organic nanotube after the freeze-drying in vacuum (10 Pa) and drying the water-containing organic nanotube in nitrogen stream, in order to substantially fully remove water.

FIG. 20 shows a comparison between respective infrared absorption spectra of the water-containing organic nanotube in the state after acetone is added thereto and then it is dried in a nitrogen stream, and the water-free organic nanotube in the state after the air-drying. As seen in FIG. 20, a shoulder is still observed around 3380 cm⁻¹ in the spectrum of the water-containing organic nanotube in the state after acetone is added thereto and then it is dried in a nitrogen stream. This shows that a quantity of water remains in the water-containing organic nanotube, and it is necessary to perform a drying operation for a longer time of period than that for the water-free organic nanotube, in order to fully dry the water-containing organic nanotube.

[Test Example 3]

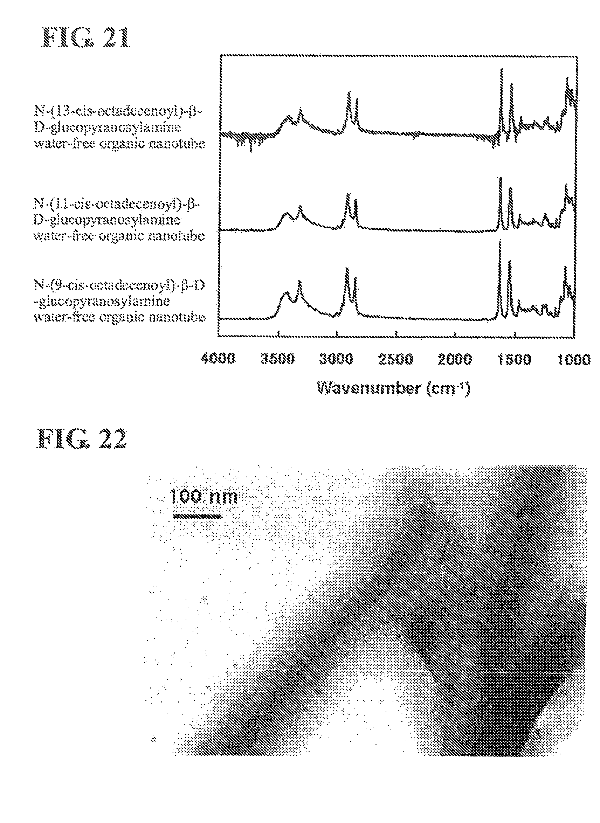

Each of the three types of water-free organic nanotubes obtained in the Examples 1 to 3 was dried at room temperature and normal pressure for one hour, and subjected to infrared spectrometry. FIG. 21 shows respective infrared absorption spectra thereof. No absorption peak of an organic solvent and water was observed in the infrared absorption spectra. Thus, it was verified that each of the three types of water-free organic nanotubes can be readily dried by the drying at room temperature and normal pressure for about one hour.

[Example 12]

98 mL of a solution of gold chloride (0.01%) was added to a 200 mL flask, and heated until it boils. 2 mL of sodium citrate (1 weight%) was added thereto under stirring, and a reaction was induced in the obtained solution for 15 minutes while continuing the heating. Then, the solution was maintained in a stationary state until a temperature thereof was returned to room temperature. A resulting colloid solution with gold nanoparticles had a red wine color. Through electron microscopic observation, it was verified that the gold nanoparticles has an average particle size of 20 nm.

The water-free organic nanotubes obtained in the Example 2 was dispersed into 10 mL of the above colloid solution with the gold nanoparticles, and the obtained dispersion liquid was stirred at room temperature and normal pressure for one hour.

[Comparative Example 2]

Gold nanoparticles were encapsulated in the water-containing organic nanotubes obtained in the Comparative Example 1, in the same manner as in the Example 12.

[Test Example 4]

The two types of organic nanotubes obtained in the Example 12 and the Comparative Example 2 were evaluated by transmission electron microscopic observation to compare between Respective encapsulation capabilities thereof.

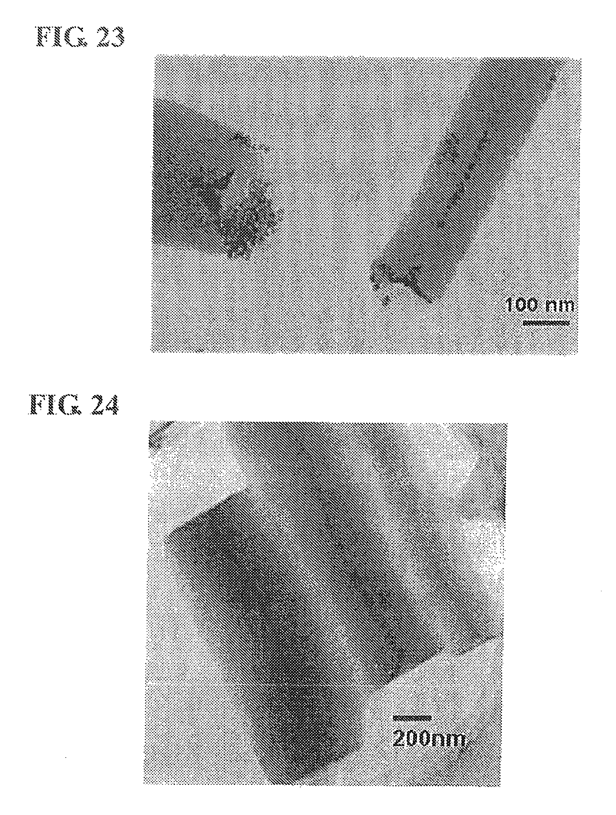

FIG. 22 shows a result of the observation of the water-free organic nanotube (Example 12), and FIG. 23 shows a result of the observation of the water-containing organic nanotube (Comparative Example 2).

As seen in FIG. 22, a hollow cylinder of the water-free organic nanotube is entirely filled with the nanoparticles. It is believed that the colloid solution with the gold nanoparticles is efficiently sucked into the water-free hollow cylinder based on a capillary phenomenon because no water or solvent exists inside the water-free organic nanotube, and thereby the functional substance is encapsulated into the water-free organic nanotube.

In contrast, as seen in FIG. 23, in the water-containing organic nanotube, gold particles are hardly observed inside a hollow cylinder thereof, and captured only at an end thereof or extremely locally.

The above result is summarized as follows. The water-free organic nanotube can be readily produced while preventing solvent (water and organic solvent) from remaining therein. This makes it possible to effectively encapsulate a functional substance therein. In contrast, in the water-containing organic nanotube, water solidly remains thereinside, and hardly removed (dried) to preclude the water-containing organic nanotube from effectively encapsulating therein.

[Example 13]

It was verified that the water-free organic nanotube obtained using an organic solvent in the Example 2 can encapsulate, in a hollow cylinder thereof, ferrosoferric oxide nanoparticles having an average particle size of 10 nm, which are used in place of the gold nanoparticles (having an average particle size of 20 nm) in the Example 12, in the same manner as that in the Example 12. FIG. 24 shows a result of encapsulation, which was obtained by transmission electron microscopic (TEM) observation.

[Example 14]

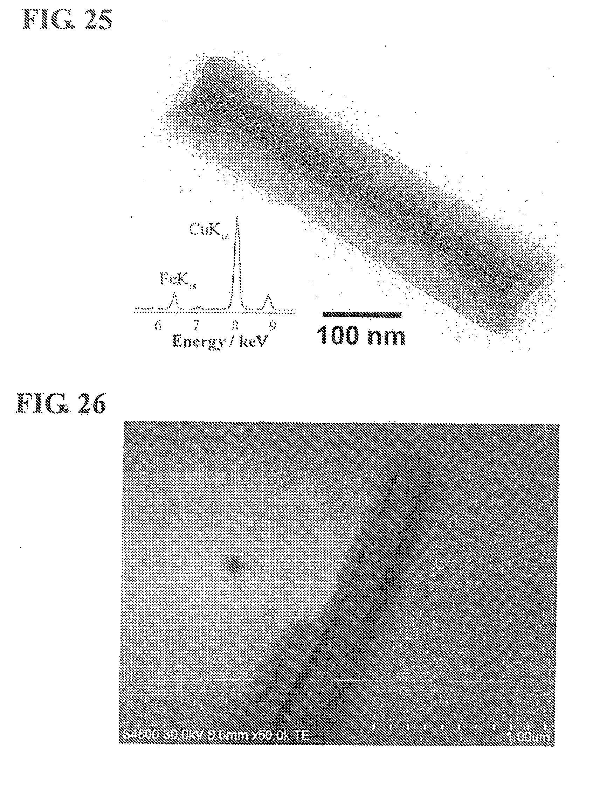

It was verified that the water-free organic nanotube obtained using an organic solvent in the Example 2 can encapsulate, in a hollow cylinder thereof, iron storage protein, ferritin, nanoparticles having an average particle size of 12 nm, which are used in place of the gold nanoparticles (having an average particle size of 20 nm) in the Example 12, in the same manner as that in the Example 12. FIG. 25 shows a result of encapsulation, which was obtained by transmission electron microscopic (TEM) observation. Based on energy dispersive X-ray analysis, iron atoms included in the protein ferritin were observed, in addition to cupper atoms included in a grid for a transmission electron microscope. Thus, black points in the photo show that the ferritin nanoparticles are efficiently encapsulated in the water-free organic nanotube.

[Example 15]

It was verified that the water-free organic nanotube obtained using an organic solvent in the Example 4 can encapsulate, in a hollow cylinder thereof, gold nanoparticles having an average particle size of 12 nm, which are used in place of the gold nanoparticles (having an average particle size of 20 nm) in the Example 12, in the same manner as that in the Example 12. FIG 26 shows a result of encapsulation, which was obtained by transmission electron microscopic (TEM) observation.

IV. INDUSTRIAL APPLICABILITY

The water-free organic nanotube of the present invention is capable of easily encapsulating a functional substance therein, and therefore usable in the fields of fine chemical industry and cosmetics, as chemicals, an encapsulating/separating material for useful biological molecules, and a drug delivery material, and in the fields of electronics/information, as microelectronic components in the form of a nanotube coated with an electrically conductive material or metal. Further, the water-free organic nanotube of the present invention is usable in the fields of medicine, analysis and chemical products, as an artificial blood vessel, a nanotube capillary and a nanoreactor utilizing a fine tube structure thereof. Thus, the present invention has a high industrial value, and can contribute to the various field.

## Claims

1. A method of producing a hollow fibrous organic nanotube, comprising the steps of:
dissolving, in an organic solvent heated up to a temperature equal to or less than a boiling point thereof, one selected from the group consisting of an N-glycoside type glycolipid represented by the following general formula 1:
G-NHCO-R¹.....
(wherein G represents a sugar residue from which a hemiacetal hydroxyl group bonded to an anomeric carbon atom of the sugar is excluded; and R¹ represents an unsaturated hydrocarbon group having 10 to 39 carbon atoms), a first peptide-lipid conjugate represented by the following general formula 2:
R²CO (NH-CHR³-CO)ₘ OH.....
(wherein: R² represents a hydrocarbon group having 6 to 24 carbon atoms; R³ represents an amino-acid side chain; and m represents an integer of 1 to 10), and a second peptide-lipid conjugate represented by the following general formula 3:
H(NH-CHR³-CO)ₘNHR² .....
(wherein: R² represents a hydrocarbon group having 6 to 24 carbon atoms; R³ represents an amino-acid side chain; and m represents an integer of 1 to 10);
slowly cooling the obtained solution;
maintaining the cooled solution undisturbed at room temperature; and
collecting from the resulting solution a hollow fibrous organic nanotube formed through self-assembling in the stationary solution, and drying the collected hollow fibrous organic nanotube, in air at room temperature or by heating under reduced-pressure.

2. A method of producing a hollow fibrous organic nanotube, comprising the steps of:
dissolving in an organic solvent one selected from the group consisting of an N-glycoside type glycolipid represented by the following general formula 1:
G-NHCO-R¹.....
(wherein G represents a sugar residue from which a hemiacetal hydroxyl group bonded to an anomeric carbon atom of the sugar is excluded; and R¹ represents an unsaturated hydrocarbon group having a 10 to 39 carbon atoms), a first peptide-lipid conjugate represented by the following general formula 2:
R²CO(NH-CHR³-CO)ₘOH.....
(wherein: R² represents a hydrocarbon group having a 6 to 24 carbon atoms; R³ represents an amino-acid side chain; and m represents an integer of 1 to 10), and a second peptide-lipid conjugate represented by the following general formula 3:
H(NH-CHR³-CO)ₘNHR² .....
(wherein: R² represents a hydrocarbon group having 6 to 24 carbon atoms; R³ represents an amino-acid side chain; and m represents an integer of 1 to 10);
concentrating the obtained solution;
maintaining the concentrated solution undisturbed at room temperature; and
collecting from the resulting solution a hollow fibrous organic nanotube formed through self-assembling undisturbed, and drying the collected hollow fibrous organic nanotube, in air at room temperature or by heating under reduced-pressure

3. The method as defined in claim 1 or 2, wherein the sugar is a monosaccharide.

4. The method as defined in claim 3, wherein the monosaccharide is glucose.

5. The method as defined in claim 1 or 2, wherein m in the general formulas 2 and 3 is 2.

6. The method as defined in claim 5, wherein the peptide in the first and second peptide-lipid conjugates is glycyl-glycine.

7. The method as defined in any one of claims 1 to 6, wherein said organic solvent contains an alcohol having a boiling point of 120°C or less, or a cyclic ether having a boiling point of 120°C or less, in an amount of at least 30 volume%.

8. A method of producing a hollow fibrous organic nanotube, comprising the steps of:
dissolving, in an organic solvent heated up to a temperature equal to or less than a boiling point thereof, one selected from the group consisting of an N-glycoside type glycolipid represented by the following general formula 1:
G-NHCO-R¹ .....
(wherein G represents a sugar residue from which a hemiacetal hydroxyl group bonded to an anomeric carbon atom of the sugar is excluded; and R¹ represents an unsaturated hydrocarbon group having 10 to 39 carbon atoms), a first peptide-lipid conjugate represented by the following general formula 2:
R²CO(NH-CHR³-CO)ₘOH.....
(wherein: R² represents a hydrocarbon group having 6 to 24 carbon atoms; R³ represents an amino-acid side chain; and m represents an integer of 1 to 10), and a second peptide-lipid conjugate represented by the following general formula 3:
H(NH-CHR³-CO)ₘNHR³ .....
(wherein: R² represents a hydrocarbon group having 6 to 24 carbon atoms; R³ represents an amino-acid side chain; and m represents an integer of 1 to 10);
adding to the obtained solution a poor solvent for the N-glycoside type glycolipid or the first and second peptide-lipid conjugates;
maintaining the mixed solution undisturbed at room temperature; and
collecting from the resulting solution a hollow fibrous organic nanotube formed through self-assembling undisturbed, and drying the collected hollow fibrous organic nanotube, in air at room temperature or by heating under reduced-pressure.

9. The method as defined in claim 8, wherein:
the organic solvent contains an alcohol having a boiling point of 120°C or less, or a cyclic ether having a boiling point of 120°C or less, in an amount of at least 30 volume%; and
the poor solvent is one or a mixture of two or more selected from the group consisting of an aromatic hydrocarbon-based solvent, a paraffin-based solvent, a paraffin chloride-based solvent, an olefin chloride-based solvent, an aromatic hydrocarbon chloride-based solvent, a straight-chain ether-based solvent, a ketone-based solvent, an ester-based solvent and a nitrogen-containing compound-based solvent.

10. A hollow fibrous organic nanotube produced by the method as defined in any one of claims 1 to 9.

11. The hollow fibrous organic nanotube as defined in claim 10, which has an average outer diameter of 70 to 500 nm, and an average inner diameter of 40 to 300 nm.

12. A hollow fibrous organic nanotube having a desired functional substance introduced thereinside by a method comprising the steps of: drying the hollow fibrous organic nanotubes as defined in claim 10 or 11; dissolving or dispersing the desired functional substance in a solvent; and dispersing the dried hollow fibrous organic nanotubes over the obtained solution or dispersion liquid.

## Patentansprüche

1. Verfahren zur Herstellung einer organischen Hohlfaser-Nanoröhre, umfassend die Schritte:
Lösen eines Mitglieds der Gruppe, die besteht aus einem Glycolipid vom N-Glycosid-Typ, dargestellt durch die folgende allgemeine Formel 1:
G-NHCO-R¹ .....
(wobei G einen Zuckerrest darstellt, welcher keine Hemiacetalhydroxylgruppe gebunden an ein anomeres Kohlenstoffatom des Zuckers, umfasst; und R¹ einen ungesättigten Kohlenwasserstoffrest mit 10 bis 39 Kohlenstoffatomen darstellt), einem ersten Peptid-Lipid-Konjugat, dargestellt durch die folgende allgemeine Formel 2:
R²CO(NH-CHR³-CO)ₘOH .....
(wobei: R² einen Kohlenwasserstoffrest mit 6 bis 24 Kohlenstoffatomen darstellt; R³ eine Aminosäure-Seitenkette darstellt; und m eine ganze Zahl von 1 bis 10 darstellt), und einem zweiten Peptid-Lipid-Konjugat, dargestellt durch die folgende allgemeine Formel 3:
H(NH-CHR³-CO)ₘNHR² ......
(wobei: R² einen Kohlenwasserstoffrest mit 6 bis 24 Kohlenstoffatomen darstellt; R³ eine Aminosäure-Seitenkette darstellt; und m eine ganze Zahl von 1 bis 10 darstellt); in einem organischen Lösungsmittel, welches auf eine Temperatur von gleich oder niedriger als ihr Siedepunkt erwärmt wird,
langsames Abkühlen der erhaltenen Lösung;
Vorhalten der abgekühlten Lösung ungestört bei Raumtemperatur; und
Sammeln einer organischen Hohlfaser-Nanoröhre, welche durch Selbstassemblierung in der stationären Lösung gebildet wurde, von der resultierenden Lösung, und Trocknen der gesammelten organischen Hohlfaser-Nanoröhre an Luft bei Raumtemperatur oder durch Erwärmen unter verringertem Druck.

2. Verfahren zur Herstellung einer organischen Hohlfaser-Nanoröhre, umfassend die Schritte:
Lösen eines Mitglieds der Gruppe, die besteht aus einem Glycolipid vom N-Glycosid-Typ, dargestellt durch die folgende allgemeine Formel 1:
G-NHCO-R¹ .....
(wobei G einen Zuckerrest darstellt, welcher keine Hemiacetalhydroxylgruppe, gebunden an ein anomeres Kohlenstoffatom des Zuckers, umfasst; und R¹ einen ungesättigten Kohlenwasserstoffrest mit 10 bis 39 Kohlenstoffatomen darstellt), einem ersten Peptid-Lipid-Konjugat, dargestellt durch die folgende allgemeine Formel 2:
R²CO(NH-CHR³-CO)ₘOH .....
(wobei: R² einen Kohlenwasserstoffrest mit 6 bis 24 Kohlenstoffatomen darstellt; R³ eine Aminosäure-Seitenkette darstellt; und m eine ganze Zahl von 1 bis 10 darstellt), und einem zweiten Peptid-Lipid-Konjugat, dargestellt durch die folgende allgemeine Formel 3:
H(NH-CHR³-CO)ₘNHR².....
(wobei: R² einen Kohlenwasserstoffrest mit 6 bis 24 Kohlenstoffatomen darstellt; R³ eine Aminosäure-Seitenkette darstellt; und m eine ganze Zahl von 1 bis 10 darstellt) in einem organischen Lösungsmittel;
Konzentrieren der erhaltenen Lösung;
Vorhalten der konzentrierten Lösung ungestört bei Raumtemperatur; und
Sammeln einer organischen Hohlfaser-Nanoröhre, welche durch Selbstassemblierung ungestört gebildet wurde, von der resultierenden Lösung, und Trocknen der gesammelten organischen Hohlfaser-Nanoröhre an Luft bei Raumtemperatur oder durch Erwärmen unter verringertem Druck.

3. Verfahren wie in Anspruch 1 oder 2 definiert, wobei der Zucker ein Monosaccharid ist.

4. Verfahren wie in Anspruch 3 definiert, wobei das Monosaccharid Glucose ist.

5. Verfahren wie in Anspruch 1 oder 2 definiert, wobei m in den allgemeinen Formeln 2 und 3 2 ist.

6. Verfahren wie in Anspruch 5 definiert, wobei das Peptid in den ersten und zweiten Peptid-Lipid-Konjugaten Glycyl-Glycin ist.

7. Verfahren wie in einem der Ansprüche 1 bis 6 definiert, wobei das organische Lösungsmittel einen Alkohol mit einem Siedepunkt von 120°C oder niedriger oder einen cyclischen Ether mit einem Siedepunkt von 120°C oder niedriger in einer Menge von mindestens 30 Volumen-% enthält.

8. Verfahren zur Herstellung einer organischen Hohlfaser-Nanoröhre, umfassend die Schritte:
Lösen eines Mitglieds der Gruppe, die besteht aus einem Glycolipid vom N-Glycosid-Typ, dargestellt durch die folgende allgemeine Formel 1:
G-NHCO-R¹ .....
(wobei G einen Zuckerrest darstellt, welcher keine Hemiacetalhydroxylgruppe, gebunden an ein anomeres Kohlenstoffatom des Zuckers, umfasst; und R¹ einen ungesättigten Kohlenwasserstoffrest mit 10 bis 39 Kohlenstoffatomen darstellt), einem ersten Peptid-Lipid-Konjugat, dargestellt durch die folgende allgemeine Formel 2:
R²CO(NH-CHR³-CO)ₘOH .....
(wobei: R² einen Kohlenwasserstoffrest mit 6 bis 24 Kohlenstoffatomen darstellt; R³ eine Aminosäure-Seitenkette darstellt; und m eine ganze Zahl von 1 bis 10 darstellt), und einem zweiten Peptid-Lipid-Konjugat, dargestellt durch die folgende allgemeine Formel 3:
H(NH-CHR³-CO)ₘNHR³ .....
(wobei: R² einen Kohlenwasserstoffrest mit 6 bis 24 Kohlenstoffatomen darstellt; R³ eine Aminosäure-Seitenkette darstellt; und m eine ganze Zahl von 1 bis 10 darstellt) in einem organischen Lösungsmittel, welches auf eine Temperatur gleich oder niedriger als ihr Siedepunkt davon erwärmt wird;
Geben eines schlechten Lösungsmittels für das Glycolipid vom N-Glycosid-Typ oder die ersten und zweiten Peptid-Lipid-Konjugate zu der erhaltenen Lösung;
Vorhalten des Lösungsgemisches ungestört bei Raumtemperatur; und
Sammeln einer organischen Hohlfaser-Nanoröhre, welche durch Selbstassemblierung ungestört gebildet wurde, von der resultierenden Lösung, und Trocknen der gesammelten organischen Hohlfaser-Nanoröhre an Luft bei Raumtemperatur oder durch Erwärmen unter verringertem Druck.

9. Verfahren wie in Anspruch 8 definiert, wobei:
das organische Lösungsmittel einen Alkohol mit einem Siedepunkt von 120°C oder niedriger oder einen cyclischen Ether mit einem Siedepunkt von 120°C oder niedriger in einer Menge von mindestens 30 Volumen-% enthält; und
das schlechte Lösungsmittel eines oder ein Gemisch von zweien oder mehreren, ausgewählt aus der Gruppe, bestehend aus einem aromatischen Lösungsmittel auf Kohlenwasserstoff-Basis, einem Lösungsmittel auf Paraffin-Basis, einem Lösungsmittel auf Paraffinchlorid-Basis, einem Lösungsmittel auf Olefinchlorid-Basis, einem aromatischen Lösungsmittel auf Kohlenwasserstoffchlorid-Basis, einem Lösungsmittel auf der Basis eines geradkettigen Ethers, einem Lösungsmittel auf Keton-Basis, einem Lösungsmittel auf Ester-Basis und einem Lösungsmittel auf der Basis einer stickstoff-haltigen Verbindung, ist.

10. Organische Hohlfaser-Nanoröhre, hergestellt durch das Verfahren wie in einem der Ansprüche 1 bis 9 definiert.

11. Organische Hohlfaser-Nanoröhre wie in Anspruch 10 definiert, welche einen mittleren äußeren Durchmesser von 70 bis 500 nm und einen mittleren inneren Durchmesser von 40 bis 300 nm aufweist.

12. Organische Hohlfaser-Nanoröhre, welche eine gewünschte funktionelle Substanz umfasst, darin eingebracht durch ein Verfahren, welches die folgenden Schritte umfasst: Trocknen der organischen Hohlfaser-Nanoröhren wie in Anspruch 10 oder 11 definiert; Lösen oder Dispergieren der gewünschten funktionellen Substanz in einem Lösungsmittel; und Dispergieren der getrockneten organischen Hohlfaser-Nanoröhren über die erhaltene Lösung oder Dispersionsflüssigkeit.

## Revendications

1. Procédé de fabrication d'un nanotube organique fibreux et creux, comprenant les étapes consistant à:
dissoudre, dans un solvant organique chauffé à une température inférieure ou égale à un point d'ébullition de celui-ci, un composé choisi dans le groupe constitué d'un glycolipide de type N-glycoside représenté par la formule générale 1 suivante:
G-NHCO-R¹ .....
(dans laquelle G représente un résidu de sucre duquel un groupe hydroxyle hémiacétal relié à un atome de carbone anomère du sucre est exclu; et R¹ représente un groupe hydrocarbure insaturé comportant de 10 à 39 atomes de carbone), d'un premier conjugué peptide-lipide représenté par la formule générale 2 suivante :
R²CO(NH-CHR³-CO)ₘOH ......
(dans laquelle: R² représente un groupe hydrocarbure comportant de 6 à 24 atomes de carbone; R³ représente une chaîne latérale d'acides aminés; et m représente un nombre entier de 1 à 10), et d'un deuxième conjugué peptide-lipide représenté par la formule générale 3 suivante:
H(NH-CHR³-CO)ₘNHR² .....
(dans laquelle: R² représente un groupe hydrocarbure comportant de 6 à 24 atomes de carbone; R³ représente une chaîne latérale d'acides aminés; et m représente un nombre entier de 1 à 10) ;
refroidir lentement la solution obtenue;
laisser reposer la solution refroidie à température ambiante; et
recueillir, à partir de la solution résultante, un nanotube organique fibreux et creux formé par auto-assemblage dans la solution stationnaire, et sécher le nanotube organique fibreux et creux recueilli, dans de l'air à température ambiante ou par chauffage sous pression réduite.

2. Procédé de fabrication d'un nanotube organique fibreux et creux, comprenant les étapes consistant à:
dissoudre dans un solvant organique un composé choisi dans le groupe constitué d'un glycolipide de type N-glycoside représenté par la formule générale 1 suivante :
G-NHCO-R¹ .....
(dans laquelle G représente un résidu de sucre duquel un groupe hydroxyle hémiacétal relié à un atome de carbone anomère du sucre est exclu; et R¹ représente un groupe hydrocarbure insaturé comportant de 10 à 39 atomes de carbone), d'un premier conjugué peptide-lipide représenté par la formule générale 2 suivante :
R²CO (NH-CHR³-CO)ₘ OH.....
(dans laquelle : R² représente un groupe hydrocarbure comportant de 6 à 24 atomes de carbone; R³ représente une chaîne latérale d'acides aminés; et m représente un nombre entier de 1 à 10), et d'un deuxième conjugué peptide-lipide représenté par la formule générale 3 suivante:
H (NH-CHR³-CO)ₘ NHR².....
(dans laquelle : R² représente un groupe hydrocarbure comportant de 6 à 24 atomes de carbone; R³ représente une chaîne latérale d'acides aminés; et m représente un nombre entier de 1 à 10);
concentrer la solution obtenue;
laisser reposer la solution concentrée à température ambiante; et
recueillir, à partir de la solution résultante, un nanotube organique fibreux et creux formé par auto-assemblage au repos, et sécher le nanotube organique fibreux et creux recueilli, dans de l'air à température ambiante ou par chauffage sous pression réduite

3. Procédé selon la revendication 1 ou 2, dans lequel le sucre est un monosaccharide.

4. Procédé selon la revendication 3, dans lequel le monosaccharide est du glucose.

5. Procédé selon la revendication 1 ou 2, dans lequel m dans les formules générales 2 et 3 vaut 2.

6. Procédé selon la revendication 5, dans lequel le peptide dans le premier et le deuxième conjugué peptide-lipide est de la glycyl-glycine.

7. Procédé selon l'une quelconque des revendications 1 à 6, dans lequel ledit solvant organique contient un alcool ayant un point d'ébullition de 120 °C ou moins, ou un éther cyclique ayant un point d'ébullition de 120 °C ou moins, dans une quantité d'au moins 30 % en volume.

8. Procédé de fabrication d'un nanotube organique fibreux et creux, comprenant les étapes consistant à:
dissoudre, dans un solvant organique chauffé à une température inférieure ou égale à un point d'ébullition de celui-ci, un composé choisi dans le groupe constitué d'un glycolipide de type N-glycoside représenté par la formule générale 1 suivante :
G-NHCO-R¹ .....
(dans laquelle G représente un résidu de sucre duquel un groupe hydroxyle hémiacétal relié à un atome de carbone anomère du sucre est exclu; et R¹ représente un groupe hydrocarbure insaturé comportant de 10 à 39 atomes de carbone), d'un premier conjugué peptide-lipide représenté par la formule générale 2 suivante :
R²CO (NH-CHR³-CO)ₘ OH.....
(dans laquelle: R² représente un groupe hydrocarbure comportant de 6 à 24 atomes de carbone; R³ représente une chaîne latérale d'acides aminés; et m représente un nombre entier de 1 à 10), et d'un deuxième conjugué peptide-lipide représenté par la formule générale 3 suivante:
H (NH-CHR³-CO)ₘ NHR³ .....
(dans laquelle: R² représente un groupe hydrocarbure comportant de 6 à 24 atomes de carbone; R³ représente une chaîne latérale d'acides aminés; et m représente un nombre entier de 1 à 10);
ajouter à la solution obtenue un solvant pauvre pour le glycolipide de type N-glycoside ou le premier et le deuxième conjugué peptide-lipide ;
laisser reposer la solution mélangée à température ambiante ; et
recueillir, à partir de la solution résultante, un nanotube organique fibreux et creux formé par auto-assemblage au repos, et sécher le nanotube organique fibreux et creux recueilli, dans de l'air à température ambiante ou par chauffage sous pression réduite.

9. Procédé selon la revendication 8, dans lequel:
le solvant organique contient un alcool ayant un point d'ébullition de 120 °C ou moins, ou un éther cyclique ayant un point d'ébullition de 120 °C ou moins, dans une quantité d'au moins 30 % en volume; et
le solvant pauvre est un composé ou un mélange de deux composés ou plus choisis dans le groupe constitué d'un solvant à base d'hydrocarbures aromatiques, d'un solvant à base de paraffine, d'un solvant à base de chlorure de paraffine, d'un solvant à base de chlorure d'oléfine, d'un solvant à base de chlorure d'hydrocarbures aromatiques, d'un solvant à base d'éther à chaîne linéaire, d'un solvant à base de cétone, d'un solvant à base d'ester et d'un solvant à base de composés contenant de l'azote.

10. Nanotube organique fibreux et creux fabriqué par le procédé selon l'une quelconque des revendications 1 à 9.

11. Nanotube organique fibreux et creux selon la revendication 10, qui a un diamètre extérieur moyen de 70 à 500 nm, et un diamètre intérieur moyen de 40 à 300 nm.

12. Nanotube organique fibreux et creux dans lequel une substance fonctionnelle souhaitée est introduite par un procédé comprenant les étapes consistant à: sécher les nanotubes organiques fibreux et creux selon la revendication 10 ou 11; dissoudre ou disperser la substance fonctionnelle souhaitée dans un solvant ; et disperser les nanotubes organiques fibreux et creux séchés sur la solution ou le liquide de dispersion obtenu(e).
